# EUROPÄISCHE PATENTSCHRIFT

(11) **EP 3 630 224 B1**
(45) Veröffentlichungstag und Bekanntmachung des Hinweises auf die Patenterteilung: **03.05.2023**
(21) Anmeldenummer: 18726798.4
(22) Anmeldetag: 23.05.2018
(51) Int. Cl.: A61M 5/142, A61M 5/315

(54) **STERILES PUMPMODUL FÜR EINE INFUSIONSPUMPE**
STERILE PUMP MODULE FOR AN INFUSION PUMP
MODULE DE POMPE STÉRILE POUR UNE POMPE A PERFUSION

(30) Priorität: 23.05.2017 DE 102017111299
(43) Veröffentlichungstag der Anmeldung: 08.04.2020
(73) Patentinhaber: B. Braun Melsungen AG, 34212 Melsungen (DE)
(72) Erfinder: FUCHS, Jürgen, 34308 Bad Emstal (DE); HARTUNG, Jürgen, 34298 Helsa (DE); STEGER, Jürgen, 34327 Körle (DE)
(74) Vertreter: Winter, Brandl - Partnerschaft mbB
(86) Internationale Anmeldenummer: PCT/EP2018/063509
(87) Internationale Veröffentlichungsnummer: WO 2018/215542

(56) Entgegenhaltungen:
- EP-A1- 2 902 052
- WO-A1-99/24098
- US-A- 4 515 591

## Beschreibung

Die vorliegende Erfindung betrifft ein Pumpmodul für eine Infusionspumpe, das eine zur wahlweisen/wechselbaren Kopplung mit der Infusionspumpe (1) ausgebildete, separate Kolben-Zylinder-Einheit zur Förderung von Fluid von einer Fluidzuleitung in eine Fluidableitung zu einem Patienten aufweist, wobei die Kolben-Zylinder-Einheit eine strömungstechnisch mit der Fluidzuleitung und der Fluidableitung verbundene Förderkammer aufweist, die durch den Zylinder, den darin in axialer Richtung translatorisch/hin-her bewegbar angeordneten Kolben und eine den Kolben gegenüber dem Zylinder abdichtende Kolbendichtung begrenzt ist. Sie betrifft des Weiteren eine Infusionspumpe zur Förderung von Fluid von einer Fluidquelle zu einem Patienten, umfassend ein solches Pumpmodul.

### Allgemeiner technischer Hintergrund

In der Infusionstechnik sind allgemein Schlauchpumpen sowie Spritzenpumpen bekannt, um einem Patienten Flüssigkeit zuzuführen. Für Anwendungen, die im Hinblick auf die Dosiergenauigkeit hohe Anforderungen stellen, werden in der Regel Spritzenpumpen verwendet, da sich mit diesen eine definierte Fluidförderung gut erzielen lässt. Bei Anwendungen, die ein größeres Fördervolumen an Fluid verlangen, sind Schlauchpumpen gut geeignet, da sie in Folge ihrer Fördertechnik weitgehend kontinuierlich und ohne diskretes Fördervolumen fördern.

### Stand der Technik

Aus dem Stand der Technik sind solche Anwendungsfälle einer Infusionspumpe bekannt, in denen relativ hohe Fördervolumina gepaart werden müssen mit einer hohen Fördergenauigkeit, was zu großen Problemen führt. Spritzenpumpen weisen nämlich den Nachteil auf, dass das mit ihnen förderbare Fluidvolumen begrenzt ist durch das Volumen der verwendeten Spritze. Schlauchpumpen hingegen können zwar relativ große Fördervolumina schaffen, besitzen jedoch den Nachteil einer geringen Fördergenauigkeit. Insbesondere bei kleinen Förderraten ist ihre Fördergenauigkeit auf Grund der üblicherweise eingesetzten Peristaltik nicht gewährleistet. Außerdem ist der Antrieb einer Schlauchpumpe in der Regel relativ groß und schwer und verbraucht durch das Walken des Pumpenschlauchs durch den Peristaltikantrieb verhältnismäßig viel Energie.

Eine speziell für solche Anwendungsfälle mit relativ hohem Fördervolumen sowie hoher Fördergenauigkeit entwickelte Infusionspumpe des Anmelders arbeitet mit einem Pumpmodul mit einer Kolben-Zylinder-Einheit als Pumpeinheit. Diese ist eingerichtet, mittels eines Antriebsmechanismus der Infusionspumpe bestehend aus einem elektrischen Antriebsmotor sowie einem Übersetzungsgetriebe zur Transformation der Rotationsbewegung des Antriebsmotors in eine Translationsbewegung/Hin-/Her-Bewegung des im Zylinder verschiebbar gelagerten Kolbens betätigt zu werden. Der Antriebsmechanismus ist vorzugsweise über eine Steuer-/Regeleinheit der Infusionspumpe gesteuert/geregelt. Das Pumpmodul umfasst außerdem eine Ventileinrichtung, welche vorzugsweise an die Steuer-/Regeleinheit angeschlossen und dafür angepasst ist, um ggf. in geregelter/gesteuerter Weise im Fall eines Pumpförderhubs eine Verbindung zwischen der Kolben-Zylinder-Einheit als Flüssigkeit-Zwischenspeicher und einem Versorgungsanschluss zu einem separaten Flüssigkeitsgroßvolumen zu unterbrechen sowie eine Verbindung zwischen der Kolben-Zylinder-Einheit und einem Patientenanschluss freizugeben und im Fall eines Pumpsaughubs die Verbindung zwischen der Kolben-Zylinder-Einheit und dem Versorgungsanschluss zu dem separaten Flüssigkeitsgroßvolumen freizugeben sowie die Verbindung zwischen der Kolben-Zylinder-Einheit und dem Patientenanschluss zu sperren.

Aus der US 3,901,231 A ist eine Infusionspumpe zum Fördern von intravenösem Fluid aus einer herkömmlichen Spritze zu einem Patienten bekannt. Die Pumpe ist anpassbar, um die Amplitude des Spritzenhubs einzustellen, um die Menge des über eine gewisse Zeitdauer an den Patienten gelieferten Fluids zu bestimmen.

Aus der US 3,985,133 ist bekannt ein Pumpmodul mit einer Kolben-Zylinder-Einheit, deren Kolben mit einem Pumpenantrieb koppelbar ist, um Fluid von einem Fluidreservoir in die Blutbahn eines Patienten zu injizieren. Aus der US 4,396,385 A ist ein ähnliches Pumpmodul bekannt, wobei der Zylinderraum mit einer einerseits am Zylinder und andererseits am Kolben angeordneten Abdeckung verschlossen ist. Bei diesem Pumpmodul ist von Nachteil, dass die Abdeckung ungeschützt vom Zylinder vorragt und ggf. einfach beschädigt werden kann, so dass Verschmutzungen in den Zylinderraum vordringen können. Ein weiterer Nachteil ist, dass dabei verwendete Rotationsventile in Bezug auf ihre Ansteuerung relativ aufwendig und wenig zuverlässig sind.

US4515591 offenbart ein Pumpmodul für eine Infusionspumpe. Es ist besonders wichtig, dass das Pumpmodul in seinen mit zu verabreichender Flüssigkeit, insbesondere Infusionsflüssigkeit oder einem Medikament, in Kontakt gelangenden Bereichen absolut steril ist und bleibt. Vorbekannte Kolbenpumpen dieser Art haben den Nachteil, dass ihr oszillierender Kolben zum Beispiel von der umgebenden Luft kontaminiert werden kann, und so Keime bis in die zu fördernde und dem Patienten zu verabreichende Flüssigkeit vordringen können.

### Kurzbeschreibung der Erfindung

Ausgehend von der vorstehend erläuterten Problematik liegt der Erfindung die Aufgabe zugrunde, die zuvor angeführten Nachteile zu beseitigen, insbesondere ein Pumpmodul sowie eine medizinische Infusionspumpe bereit zu stellen, das bzw. die bekannte Systeme ersetzen kann, universell insbesondere bei Anwendungen mit großer Volumenförderung und hoher Fördergenauigkeit einsetzbar ist und außerdem in Bereichen, die mit einer einem Patienten zu verabreichender Flüssigkeit in Kontakt gelangen, absolut steril ist und während einer Verwendung bleibt. Die Sterilität soll insbesondere sicher gewährleistet sein. Außerdem soll das Pumpmodul besonders anwenderfreundlich in der Pumpe montierbar sein, wobei weder bei der Handhabung noch beim Einsatz noch bei der Lager des Pumpmoduls dessen Sterilität gefährdet wird. Vorzugsweise soll die Baugröße im Vergleich zu bekannten Systemen gering sein. Es bzw. sie soll des Weiteren einfach und kostengünstig sein und vorzugsweise mittels eines Akkus betrieben werden können.

Diese Aufgabe wird nach der Erfindung gelöst durch ein Pumpmodul, insbesondere ein Infusions-/Spritzenpumpmodul, für eine medizintechnische Infusionspumpe zur Förderung von Fluid von einer Fluidquelle zu einem Patienten mit den Merkmalen des Patentanspruchs 1 sowie durch eine Infusionspumpe mit den Merkmalen des Anspruchs 12. Vorteilhafte Ausgestaltungen der Erfindung sind Gegenstand der Unteransprüche.

Die Aufgabe wird insbesondere gelöst durch ein Pumpmodul für eine Infusionspumpe, das eine mit der Infusionspumpe koppelbare Kolben-Zylinder-Einheit, insbesondere eine zur wahlweisen/wechselbaren Kopplung mit der Infusionspumpe (1) ausgebildete, separate Kolben-Zylinder-Einheit zur Förderung von Fluid von einer Fluidzuleitung in eine Fluidableitung zu einem Patienten aufweist, wobei die Kolben-Zylinder-Einheit eine strömungstechnisch mit der Fluidzuleitung und der Fluidableitung verbundene Förderkammer aufweist, die durch den Zylinder, den darin in axialer Richtung, insbesondere in axialer Richtung der Kolben-Zylinder-Einheit, translatorisch/hin-her bewegbar angeordneten Kolben und eine den Kolben gegenüber dem Zylinder abdichtende Kolbendichtung begrenzt ist, wobei der von der Kolbendichtung bei der Hin-her-Bewegung des Kolbens überlaufene Wandabschnitt des Zylinders gegenüber der Umgebung steril abgedichtet ist, insbesondere wobei die Kolbendichtung einen sich längs des Kolbens erstreckenden und diesen relativ bewegbar umgebenden Hüllenabschnitt aufweist, der relativ zum Kolben beweglich ausgebildet ist und lose zwischen einem Umfangs-/Wandabschnitt des Zylinders und dem Kolben gehalten und an einem der Förderkammer abgewandten Ende oder Endbereich des Zylinders an diesem dichtend fixiert ist, derart, dass der von der Kolbendichtung bei der Hin-her-Bewegung des Kolbens überlaufene Umfangs-/ Wandabschnitt des Zylinders gegenüber der Umgebung steril abgedichtet ist. Sie wird außerdem gelöst durch eine Infusionspumpe mit einem Pumpmodul nach der Erfindung, insbesondere nach der vorliegenden Beschreibung und den angehängten Ansprüchen. Durch die Erfindung wird gegenüber dem Stand der Technik der Vorteil und Effekt bewirkt, dass das oszillierende Pumpelement, hier in Form der Kolben-Zylinder-Einheit, insbesondere in den Bereichen, die mit dem einem Patienten zu verabreichenden Fluid in Kontakt gelangen, durch das Dichtelement gegen Verkeimung geschützt ist. Bei diesen Bereichen handelt es sich insbesondere um die Abschnitte der Zylinderwand, die von der Kolbendichtung beim Kolbenhub überstrichen werden. Bei der Kolben-Zylinder-Einheit handelt es sich vorzugsweise um eine leichtgängige Einheit, um Betätigungskräfte gering zu halten und eine hohe Fördergenauigkeit zu erzielen.

Vorteilhafte Ausführungsformen der Erfindung sind in den Unteransprüchen beansprucht und werden nachfolgend näher erläutert.

Nach einer Ausführungsform der Erfindung kann der Hubraum, der sich auf der der Förderkammer gegenüberliegenden Seite der Kolbendichtung befindet, insbesondere der gesamte Hubraum, mittels eines darin, also innerhalb des Hubraums, angeordneten elastischen Dichtelements gegenüber der Umgebung steril abgedichtet sein. Das Dichtelement kann insbesondere eine den proximalen Hubraum gegenüber der Umgebung steril abdichtende Membran sein, die in Folge ihrer Elastizität die relativen Lageänderungen zwischen Kolben und Zylinder durch die Hin-Her-Bewegung des Kolbens ausgleichen kann. Es kann außerdem einerseits am proximalen Ende des Kolbens und andererseits am proximalen Ende des Zylinders angeordnet und/oder damit jeweils fest verbunden sein. Proximal bedeutet in diesem Zusammenhang auf Seiten der Pumpe, distal auf Seiten der Förderkammer. Bei einer bevorzugten Ausführungsform ist das Dichtelement derart ausgebildet und am Zylinder und am Kolben angeordnet, dass es, insbesondere unabhängig von der jeweiligen Position des Kolbens im Zylinder, nicht über das proximale Ende des Zylinders ragt. Auf diese Weise ist das Dichtelement geschützt innerhalb des Zylinder angeordnet, so dass versehentliche Beschädigungen bei Lagerung, Montage und/oder Einsatz des Pumpmoduls sicher vermieden werden können. Dies trägt dazu bei, dass die Sterilität der Bereiche des Pumpmoduls, die mit einem einem Patienten zu verabreichenden Fluid in Kontakt gelangen, sicher gewährleistet werden kann.

Im Rahmen der Erfindung kann außerdem das Dichtelement einen als Faltenbalg ausgebildeten und sich insbesondere in axialer Richtung erstreckenden Wandabschnitt aufweisen. Dieser kann vorzugsweise innerhalb des Zylinders angeordnet sein, so dass er vor Beschädigungen geschützt ist. Ein solcher Faltenbalg nimmt wenig Raum ein und unterliegt außerdem beim Betrieb des Pumpmoduls nur relativ geringen Belastungen infolge der ihm auferlegten Verformung, so dass das Pumpmodul klein und robust ausgebildet sein kann.

Eine besonders robuste Ausführungsform der Erfindung sieht vor, dass das Dichtelement an seiner von Kolben abgewandten Seite einen insbesondere ringförmigen Koppelabschnitt zum dichtenden Anordnen an und/oder zum dichtenden Verbinden mit dem Zylinder aufweist. Diese kann mittels einer Bördelung mit dem Zylinder verbunden sein, insbesondere dicht verbunden sein. Die Bördelung kann zum Beispiel durch Bördeln eines proximalen Endabschnitts des Zylinders, insbesondere der Zylinderwandung, ausgebildet sein. Alternativ oder zusätzlich kann das Dichtelement mit seiner dem Kolben zugewandten Seite dichtend mit dem Kolben verbunden sein. Es kann insbesondere stirnseitig des Kolbens mit diesem verbunden sein. Beispielsweise ist das Dichtelement stoffschlüssig mit dem Kolben verbunden. Es kann insbesondere einstückig mit dem Kolben ausgebildet sein, zum Beispiel indem der Kolben und das Dichtelement mittels 2-K-Technologie hergestellt sind. Der Koppelabschnitt kann insbesondere in Form einer Dichtplatte ausgebildet sein.

Nach einer besonders anwenderfreundlichen Ausführungsform kann der Kolben eine Koppelstruktur zum lösbaren Kuppeln mit einem entsprechenden Kuppelelement des Antriebsmechanismus der Infusionspumpe aufweisen. Diese kann mit besonderem Vorteil radial innerhalb des Dichtelements, insbesondere radial innerhalb des als Faltenbalg ausgebildeten Wandabschnitts des Dichtelements, ausgebildet sein. Zum Beispiel kann die Koppelstruktur ein Sackloch, insbesondere ein zentrales Sackloch, aufweisen, das in axialer Richtung in die proximale Stirnfläche des Kolbens eingebracht ist. Dies ermöglicht eine besonders kurze Baulänge des Kolbens und damit des Pumpmoduls. Um eine besonders einfache Kopplung von Pumpmodul und Infusionspumpe zu ermöglichen, kann die Koppelstruktur eine innenliegende Raststruktur zur rastenden Aufnahme einer Kolbenstange des Antriebsmechanismus aufweisen. Der Kolben kann mittels der Koppelstruktur insbesondere an einer Kolbenstange der Infusionspumpe angeordnet oder anzuordnen sein. Das Koppelelement kann zum Beispiel in Form einer Steckkupplung ausgebildet sein und beim bestimmungsgemäßen Anordnen des Pumpmoduls in der Infusionspumpe automatisch oder zwangsläufig mit dem Antrieb gekoppelt werden. Vorzugsweise weist die Kupplung Raststrukturen auf, die beide Kuppelelemente aneinander halten und insbesondere ein für einen Anwender hörbares und/oder fühlbares Verrasten der beiden Kuppelelemente ermöglichen.

Eine Ausführungsform der Erfindung ist dadurch gekennzeichnet, dass die Kolben-Zylinder-Einheit eine proximale Kolbendichtung und eine distale Kolbendichtung aufweist. Diese dichten beide zwischen dem Kolben und dem Zylinder und gewährleisten die Sterilität der Förderkammer. Es liegt insbesondere im Rahmen der Erfindung, dass das Pumpmodul nur proximale und distale Kolbendichtungen und kein mit dem Kolben und dem Zylinder verbundenes Dichtelement aufweist. Vorzugsweise sind die proximale Kolbendichtung und die distale Kolbendichtung zueinander planparallel angeordnet. Alternativ oder zusätzlich ist der Abstand in Richtung der Längsachse zwischen der proximalen Kolbendichtung und der distalen Kolbendichtung größer als der Förderhub der Kolben-Zylinder-Einheit / des Kolbens im Zylinder. Vorzugsweise ist der Abstand zwischen den beiden Kolbendichtungen mindestens 1 mm bis 3 mm, insbesondere 2 mm größer als der Hub der Kolbenpumpe (und damit als der Hub des Kolbens im Zylinder). Dadurch ist gewährleistet, dass der mit dem zu verabreichenden Fluid in Kontakt gelangende Bereich immer absolut steril ist. Die proximale Kolbendichtung und/oder die distale Kolbendichtung kann bzw. können einstückig mit dem Kolben ausgebildet sein, zum Beispiel indem sie mittels 2-K-Technologie an den Kolben angeformt ist bzw. sind. Die Kolbendichtung(en) kann/können grundsätzlich im Rahmen der Erfindung insbesondere in Form einer flexiblen Dichtlippe bzw. Dichtlippen ausgebildet sein. Ein besonderer Vorteil der vorbeschriebenen Ausführungsform mit einer proximalen und einer distalen Kolbendichtung ist, dass die Kolben-Zylinder-Einheit besonders leichtgängig ist und Betätigungskräfte gering sind, so dass eine hohe Fördergenauigkeit und -effizienz erzielt werden kann. Man kann auch sagen, dass der Kolben und der Abstand der beiden Kolbendichtungen in axialer Richtung länger als der maximale Füllstand des Zylinders sind, wodurch vermieden werden kann, dass sich Umgebungsluft, die sich naturgemäß außerhalb des Zylinders befindet, mit dem im Zylinder befindlichen Medium vermischt oder Bereiche der Kolben-Zylinder-Einheit kontaminieren kann, die mit dem zu fördernden Fluid in Kontakt gelangen. In der Kombination aus zwei Kolbendichtungen / Dichtlippen und Kolben / Zylinderlänge ergibt sich eine wirksame Barriere gegen eindringende Keime. Ein weiterer Vorteil ist, dass diese Ausführungsform mit zwei Kolbendichtungen relativ hohen Druckdifferenzen bei der Fluidförderung standhalten kann, ohne dass zu förderndes Fluid und/oder Luft die Dichtung / Dichtlippe zwischen Kolben und Zylinder überwinden kann. Man kann daher auch sagen, dass der Kolben jeweils eine Dichtung / Dichtlippe zum Abdichten gegen Überdruck und eine Dichtung / Dichtlippe zum Abdichten gegen Unterdruck aufweist. So ist eine Unterdruckbewegung und eine Überdruckbewegung des Kolbens abgedichtet, um eine Sterilität eines Fluidraums / Dosierraums, das heißt des Raums, in dem die sich die zu fördernde Flüssigkeit im Zylinder aufhält, zu gewährleisten. So erfüllt die Pumpe die hohen Anforderungen hinsichtlich der Sauberkeit in medizinischem Umfeld. Der Kolben hat vorzugsweise einen im Wesentlichen runden Querschnitt, so dass hieraus etwa ringförmige Kolbendichtungen resultieren.

Bei einer besonders lagerfreundlichen Ausführungsform der Erfindung, die eine relativ lange Lagerung des Pumpmoduls ohne wesentliche Beeinträchtigung der Dichtwirkung der Kolbendichtung(en) ermöglicht, ist der Zylinder mit einer der Anzahl an Kolbendichtungen entsprechenden Anzahl an Ringnuten versehen, also im Falle nur einer Kolbendichtung auf seiner dem Kolben zugewandten Innenfläche mit nur einer umlaufenden Ringnut, zur Aufnahme der Kolbendichtung in einer Lagerstellung/Ruhestellung darin. Im Falle eines Kolbens mit einer proximalen und einer distalen Kolbendichtung ist der Zylinder auf seiner dem Kolben zugewandten Innenfläche mit einer proximalen umlaufenden Ringnut und einer distalen umlaufenden Ringnut versehen, deren Abstand in Längsrichtung voneinander gleich dem Abstand der proximalen Kolbendichtung und der distalen Kolbendichtung in Längsrichtung voneinander ist. Die Ringnut(en) ist/sind vorzugsweise so angeordnet, dass sie sich außerhalb des Arbeitsbereichs des Kolbens befinden.

Eine Ausführungsform der Erfindung ist dadurch gekennzeichnet, dass der Zylinder an seinem distalen Ende einen Zylinderkopf aufweist, in dem zumindest ein Fluiddurchlass ausgebildet ist, um die Förderkammer strömungstechnisch mit der Fluidzuleitung und/oder mit der Fluidableitung zu verbinden. Der Zylinderkopf kann insbesondere die Förderkammer distal begrenzen. Eine besonders anwenderfreundliche und einfach mit der Infusionspumpe zu koppelnde Ausführungsform der Erfindung sieht vor, dass distal an dem Zylinder, insbesondere an dessen Zylinderkopf, ein Griffabschnitt oder Griffstück angeordnet ist, zur Handhabung des Pumpmoduls beim Koppeln und Entkoppeln mit der Infusionspumpe.

Nach einer Ausführungsform der Erfindung kann die Kolben-Zylinder-Einheit mit einem ersten Schlauchabschnitt als Zulaufleitung / Fluidzuleitung und einem zweiten Schlauchabschnitt als patientenseitige Auslaufleitung / Fluidableitung versehen sein. Zumindest einer der oder beide Schlauchabschnitte können fest mit der Kolben-Zylinder-Einheit, insbesondere mit dem Zylinderkopf verbunden sein, beispielsweise einstückig, kraftschlüssig oder stoffschlüssig, zum Beispiel durch Anspritzen im Falle von Kunststoffteilen.

Die Fluidzuleitung bzw. der erste Schlauchabschnitt kann am der Kolben-Zylinder-Einheit gegenüberliegenden Ende mit einem Luer-Lock-Kupplungsstück versehen sein. Dieses kann insbesondere als Luer-Lock-Innenkegel ausgebildet sein. Alternativ oder zusätzlich kann die Fluidableitung bzw. der zweite Schlauchabschnitt am der Kolben-Zylinder-Einheit gegenüberliegenden Ende mit einem Luer-Lock-Kupplungsstück versehen sein. Dieses kann insbesondere als Luer-Lock-Außenkegel ausgebildet sein. Dies ermöglicht eine besonders einfache zu betätigende Möglichkeit zur Verbindung des Pumpmoduls bzw. dem darin aufgenommenen Flüssigkeitsleitungssystem mit einer extrakorporalen Leitung oder Leitungssystem. Die Erfindung lässt sich damit ohne weiteres und in bekannter Weise zusammen mit bestehenden und weitverbreiteten medizintechnischen Einrichtungen verwenden. Alternativ zu einem Luer-Lock-Anschluss kann eine am Patienten angeschlossene Tropfkammer vorgesehen sein oder der zweite Schlauchabschnitt ist als Sackleitung mit einem verschlossen Ende ausgebildet und kann mit einem Einsteckdorn angeschlossen werden. Zusätzlich oder alternativ bietet sich auch das Anordnen eines Spikes / Einstechdorns an, um die Flexibilität im Anwendungsgebiet bzw. die Kompaktheit der Pumpe zu erhöhen.

Nach einer Ausführungsform kann das Pumpmodul eine Schiebeklemme aufweisen, die derart einstellbar, das heißt verschiebbar ist, dass sie einen Fluidfluss von oder zur Kolben-Zylinder-Einheit, insbesondere in dem ersten und/oder dem zweiten Schlauchabschnitt, wahlweise freigibt oder sperrt. So ist die Schiebeklemme derart einstellbar, dass sich nach der Inbetriebnahme des Pumpmoduls keine Luftblasen bilden. Die Schiebklemme ist vorzugsweise auf den Griffabschnitt oder das Griffstück des Pumpmoduls aufclipsbar, sodass diese leicht aus einem anderen Material herstellbar ist als das restliche Pumpmodul.

Eine vorteilhafte Ausführungsform der Erfindung zeichnet sich dadurch aus, dass an einem der Schiebeklemme abgewandten Ende des Pumpmoduls bzw. des Griffabschnitts / -stücks eine Hülse / ein Ring angeordnet ist. Diese Hülse ist dazu in der Lage, die Fluidzuleitung und/oder die Fluidableitung bzw. den ersten und/oder den zweiten Schlauchabschnitt zu fixieren und somit eine sichere Kopplung einer Infusionsleitung mit dem Pumpmodul zu ermöglichen. Sie dient demnach als Haltering, durch den die Infusionsleitung einfädelbar ist.

Die Kolben-Zylinder-Einheit kann insbesondere dafür angepasst sein, mittels eines Antriebsmechanismus, insbesondere mittels eines Antriebsmechanismus der Infusionspumpe nach der Erfindung, beispielsweise bestehend aus einem elektrischen Antriebsmotor sowie einem Übersetzungsgetriebe zur Transformation der Rotationsbewegung des Antriebsmotors in eine Translationsbewegung/Hin-/Her-Bewegung des im Zylinder verschiebbar gelagerten Kolbens betätigt zu werden. Der Antriebsmechanismus kann weiter zum Beispiel über eine Steuer-/Regeleinheit der Infusionspumpe gesteuert/geregelt sein. Außerdem kann eine Ventileinrichtung vorgesehen sein, zum Beispiel die Infusionspumpe eine Ventileinrichtung aufweisen, die an die Steuer-/Regeleinheit angeschlossen und eingerichtet ist, um (ggf. in geregelter/gesteuerter Weise) im Fall eines Pumpförderhubs eine Verbindung zwischen der Kolben-Zylinder-Einheit als Flüssigkeit-Zwischenspeicher und einem Versorgungsanschluss zu einem separaten Flüssigkeitsgroßvolumen zu unterbrechen sowie eine Verbindung zwischen der Kolben-Zylinder-Einheit und einem Patientenanschluss freizugeben und im Fall eines Pumpsaughubs die Verbindung zwischen der Kolben-Zylinder-Einheit und dem Versorgungsanschluss zu dem separaten Flüssigkeitsgroßvolumen freizugeben sowie die Verbindung zwischen der Kolben-Zylinder-Einheit und dem Patientenanschluss zu sperren.

Im einfachsten Fall hat die Ventileinrichtung passiv betätigbare Ventilelemente, d.h. die Ventileinrichtung hat bevorzugt zwei Rückschlagventile, welche in (separaten, als Einweg-Artikel ausgebildeten) Verbindungsleitungen zum Patientenanschluss und zum Flüssigkeitsgroßvolumen untergebracht sind, die in die Infusionspumpe einlegbar sind. Dies hat den Vorteil, dass die Ventileinrichtung besonders preisgünstig herstellbar ist und sich damit als Einweg-Artikel zusammen mit dem gesamten Flüssigkeitsleitungssystem entsorgt werden können.

Alternativ kann vorgesehen sein, die Ventileinrichtung mit aktiv betätigbaren Ventilelementen, vorzugsweise zwei Schlauchquetschen/Pressen zu versehen, die auf flexibel deformierbare Abschnitte der beiden Verbindungsleitungen für deren dichtendes Abdrücken einwirken können und die bevorzugt in der Infusionspumpe (oder Pumpenmodul) untergebracht sind. Im Konkreten hat demnach das vorzugsweise als Einweg-Artikel ausgebildete Flüssigkeitsleitungssystem einen ersten, vorzugsweise elastisch verformbaren Schlauchabschnitt zur Fluidzufuhr (aus dem Flüssigkeitsgroßspeicher in den Zwischenspeicher) und einen zweiten, vorzugsweise elastisch verformbaren Schlauchabschnitt zur Fluidabfuhr (aus dem Zwischenspeicher in Richtung hin zum Patienten), wobei die beiden Schlauchabschnitte für ein Einsetzen in die Infusionspumpe sowie für ein Anschließen an die Kolben-Zylinder-Einheit (Flüssigkeitszwischenspeicher) zum Ansaugen von Fluid aus dem ersten Schlauchabschnitt in den Flüssigkeitszwischenspeicher aufweist. Die Schlauchquetschen/Pressen können bevorzugt als Stempel/Stößel oder als Scherenklemmen oder dergleichen mechanische Klemmmittel ausgebildet sein, die beweglich in der Infusionspumpe gelagert sind (sind damit Bestandteil der Infusionspumpe) und von jeweils einem Antrieb betätigt werden können, der an die Steuer-/Regeleinheit angeschlossen ist. Diese Variante ermöglich ein kontrolliertes und damit sicheres Öffnen und Sperren der jeweiligen Flüssigkeits-/Fluidleitungen und auch ein preisgünstiger herstellbares Flüssigkeitsleitungssystem. Kennzeichnendes Merkmal hierbei ist, dass die beiden im Einlass und Auslass befindlichen Schlauchquetschen/Pressen derart mechanisch miteinander gekoppelt sind, dass mindestens eine Seite den Schlauch sicher abdrückt. Damit beim Wechsel zwischen Einlass und Auslass keine undichte Position entsteht werden hierzu die Schlauchquetschen/Pressen mit z.B. Federn vorgespannt, so dass diese die ansonsten undichten Positionen überbrücken. Alternativ kann die Steuerzeit der Ventile auch über z.B. Kurvenscheiben oder durch getrennte Ansteuerungen mittels Motoren erfolgen

Über die Steuereinheit, die zum Beispiel in Form einer motorisch betriebenen Ventilsteuerung realisiert sein kann, kann der Schlauch im Auslauf, also der zweite Schlauchabschnitt, gesperrt, vorzugsweise abgedrückt werden, wenn die Kolben-Zylinder-Einheit / Spritze aufgezogen wird. Zudem kann der Schlauch im Zulauf, also der erste Schlauchabschnitt, gesperrt, vorzugsweise abgedrückt werden, wenn die Kolben-Zylinder-Einheit / Spritze ausgedrückt wird. Bezeichnend ist, dass die Kolben-Zylinder-Einheit / die Spritze wie eine Kolbenpumpe angesteuert werden kann. Insgesamt ist es durch die Erfindung möglich, bei Infusionsanwendungen im weitesten Sinne große Fluidmengen / Volumina mit der hohen Präzision einer Kolben-/Spritzenpumpe zu fördern. Die hohe Präzision ist dadurch bedingt, dass die Förderungsrate / Fördermenge aufgrund der mit der Kolben-Zylinder-Einheit durchgeführten Förderung sehr präzise ist. Änderungen des Fördervolumens, also des Volumens der Zylinderkammer, können in einem weiten Förderratenbereich genau eingestellt und angesteuert werden. Leckageströme können durch diese Bauart im Wesentlichen vollständig vermieden werden. Der Nachteil der bei Kolbenpumpen üblicherweise vorliegenden eingeschränkten Fördermenge wird im Sinne der Erfindung dadurch behoben, dass die Kolben-Zylinder-Einheit fortlaufend alternierend gefüllt und entleert wird, was durch die erfindungsgemäße Steuerung der Zu- und Abströmungen zur bzw. von der Kolben-Zylinder-Einheit ermöglicht wird. Eine Möglichkeit, eine weitgehend konstante Förderrate zu bewirken, besteht in der Kombination zweier erfindungsgemäßer Pumpen oder darin, die Pumpe mit zwei Kolben-Zylinder-Einheiten und diesen jeweils zugeordneten Steuereinheiten zu versehen, die phasenverschoben betrieben werden.

Eine bevorzugte Ausführungsform der Infusionspumpe nach der Erfindung ist dadurch gekennzeichnet, dass die Steuereinheit und der Antrieb an/in einem Gehäuseteil der Infusionspumpe angeordnet sind. Das Pumpmodul kann insbesondere anwenderseitig austauschbar an dem Gehäuseteil anzuordnen sein. Dabei ist es vorzugsweise ohne Nutzung besonderer Werkzeuge oder Vorrichtungen in der Pumpe anzuordnen und/oder aus dieser zu entfernen. Das erfindungsgemäße Pumpmodul kann insbesondere als Einmalartikel ausgebildet und für einzige Verwendung in der Pumpe bestimmt sein. Dies ist im Hinblick auf Sterilbedingungen besonders vorteilhaft und anwenderfreundlich. Es kann zum Beispiel in Form einer Einmalspritze realisiert sein, mit deren Fördervolumenöffnung die beiden Schlauchabschnitte strömungstechnisch verbunden sind. Dies kann derart realisiert sein, dass die beiden Schlauchabschnitt eine fortlaufende Fluidleitung ausbilden, die in einem Bereich zwischen den Deformationsstellen mit einer Abzweigung zum Anschließen der Kolben-Zylinder-Einheit, insbesondere einer Einmalspritze, versehen ist. Alternativ kann die Kolben-Zylinder-Einheit / die Einmalspritze zwei Strömungsöffnungen aufweisen, einen Ausgang und einen Eingang, die jeweils strömungstechnisch mit dem entsprechenden Schlauchabschnitt verbunden werden.

Es ist von besonderem Vorteil, wenn der Antrieb der Kolben-Zylinder-Einheit ein Linearantrieb/-motor ist, der in der Bewegungsachse des Kolbens angeordnet ist. Besonders bevorzugt ist, wenn der Antrieb derart angeordnet und ausgebildet ist, dass vom Antrieb auf den Kolben wirkende Kräfte zentral und in Achsrichtung eingeleitet werden. Dabei ist von besonderem Vorteil, dass auf den Kolben wirkende Kräfte minimiert werden können (im Vergleich zu einer dezentralen Krafteinleitung) und derart nur eine geringe Energie zur Betätigung der Kolben-Zylinder-Einheit erforderlich ist. In der Folge kann der Antrieb und damit die Pumpe klein ausgebildet sein, was zu Einsparungen hinsichtlich Gewicht, Kosten und Bauraum führt. Außerdem kann durch die zentrale Einleitung von Betätigungskräften in den Kolben eine hohe Präzision erzielt werden. Die Verwendung einer kleinen Spritze als Kolben-Zylinder-Einheit ermöglicht eine Anordnung eines Linearantriebs unmittelbar in der Achse des Spritzenkolbens, wodurch erreicht wird, dass keine die Genauigkeit beeinflussenden Querkräfte auf den Spritzenkolben wirken. Des Weiteren erzeugt ein kleiner Querschnitt der Spritze nur geringe Kräfte, was eine Verwendung eines einfachen kostengünstigen Antriebs, zum Beispiel eines Linear-Schrittmotors, ermöglicht. Die Baugröße der Pumpe kann dadurch deutlich geringer ausfallen als bei bekannten Infusionspumpen.

Die erfindungsgemäße Pumpe kann außerdem einen zweiten Antrieb, insbesondere Linearantrieb/-motor, für die Steuereinheit aufweisen. Die beiden Antriebe für die Kolben-Zylinder-Einheit und die Steuereinheit können steuerungstechnisch miteinander gekoppelt sein, derart, dass die vorstehend beschriebene Steuerungsfunktion durch Abquetschen der beiden Schlauchabschnitte erfolgt.

Nach einer weiteren Ausführungsform kann die Pumpe eine Aufnahme für das Pumpmodul aufweisen. Diese kann beispielsweise in Form einer Ausnehmung in einem Pumpengehäuse ausgebildet sein und insbesondere mittels eines Verschlusses (zum Beispiel in Form einer schwenkbar an der Pumpe, insbesondere an deren Gehäuse, angeordneten Verschlussklappe) zu verschließen sein. Der Verschluss kann insbesondere in einer die Aufnahme verschließenden Stellung über eine Verriegelungseinheit mit dem Gehäuseteil verriegelbar sein. Der Verschluss, die Aufnahme und das Pumpmodul können derart aufeinander abgestimmt sein, dass ein Schließen (und ggf. Verriegeln) des Verschlusses nur möglich ist, wenn das Pumpmodul in bestimmungsgemäßer Weise und korrekt angeordnet und angeschlossen ist. Derart kann einem Nutzer ein Feedback im Hinblick auf eine fehlerfreie Einrichtung und Nutzung der Pumpe gegeben werden, was in vorteilhafter Weise die Patientensicherheit erhöht. Außerdem kann durch ein Schließen der Verschlussklappe ein automatisches Kuppeln der Kolben-Zylinder-Einheit mit dem Antrieb bewirkt werden, was eine besonders einfache und sichere Bedienung darstellt. Ein Gehäuse der Pumpe kann insbesondere aus einem Gehäuseunterteil, an dem die gesamte Mechanik und Elektronik angeordnet und gehalten sein kann, sowie aus einem Gehäuseoberteil, das insbesondere ein Display und diversen Schaltelemente aufweisen kann, bestehen. Im Inneren des Gehäuses kann sich ein Verschlussmechanismus für die Verschlussklappe befinden.

Eine Ausführungsform der Infusionspumpe ist dadurch gekennzeichnet, dass die Steuereinheit eine motorisch angetriebene Kipphebeleinheit hat. Diese kann derart ausgebildet sein und wirken, dass sie in einer ersten Kippstellung den ersten Schlauchabschnitt zusammenquetscht und den zweiten Schlauchabschnitt freigibt und in einer zweiten Kippstellung den zweiten Schlauchabschnitt zusammenquetscht und den ersten Schlauchabschnitt freigibt.

Die Steuereinheit kann insbesondere einen schwenkbar an der Kipphebeleinheit angeordneten Druckstempel aufweisen. Sie kann vor allem einen mit dem ersten Schlauchabschnitt zusammenwirkenden Einlassstempel und/oder einen mit dem zweiten Schlauchabschnitt zusammenwirkenden Auslassstempel umfassen. Zumindest ein Druckstempel kann mittels einer Vorspanneinheit, insbesondere mittels einer Druckfeder, in eine den jeweiligen Schlauchabschnitt freigebenden, insbesondere nicht kontaktierende, Position vorgespannt sein. Auf diese Weise kann bewirkt werden, dass sich die Druckstempel nicht im Bereich des entsprechenden Schlauchabschnitts befinden, wenn die Pumpe nicht in Betrieb ist. Dies ermöglicht ein besonders anwenderfreundliches Wechseln, Einsetzen und Entfernen des erfindungsgemäßen Pumpmoduls.

Da der Antrieb / die Antriebe der Pumpe klein ausgelegt sein können, ist es in vorteilhafter Weise möglich, dass die Pumpe eine Energiespeichereinheit, insbesondere einen Akku, umfasst und ohne direkten Netzanschluss betrieben werden kann. Die Energiespeichereinheit kann zum Beispiel ein üblicher Lithium-Akku sein, der über einen standardisierten Steckverbinder beispielsweise in Form einer USB-Schnittstelle geladen werden kann. Diese kann außerdem genutzt werden, um Daten in eine Pumpensteuerung ein- und/oder auszulesen.

Zusammenfassend kann man sagen, dass die Erfindung ein Infusionspumpsystem mit der Genauigkeit einer Spritzenpumpe und dem Fördervolumen oder Volumenvorrat einer Schlauchpumpe bei deutlich geringerem Energiebedarf, reduzierter Baugröße und geringen Herstellungskosten ermöglicht. Die stellt insbesondere ein Pumpmodul für eine Kolbenpumpe zur Verfügung, welches über ein hubgesteuertes Kolbensystem verfügt. Das Pumpmodul/Pumpelement selbst ist in besonders vorteilhafter Weise in dem Bereich, wo sich Infusionsflüssigkeit befindet, absolut steril. Durch die Erfindung können insbesondere die folgenden Vorteile erzielt werden:
- "One fits all": eine große Bandbreite an Infusionstherapien (nahezu alle bekannten) können mit nur einer einzigen Pumpe ausgeführt werden.
- Kostengünstige und kleine Infusionspumpe
- Die Pumpe kann an beliebigen Orten (überall) eingesetzt werden, insbesondere ohne Netzanschluss (mobil lange Akkulaufzeit), was sie besonders für Außeneinsätze oder für Einsätze in weniger entwickelten Gebieten qualifiziert.
- Die Pumpe ermöglicht eine genauere Dosierung der Infusion als übliche Infusionspumpen.
- Feste Bindung des insbesondere als Einmalartikel ausgebildeten Pumpmoduls an die Pumpe
- Das Pumpmodul ist mit verschiedenen Arten von Infusionsleitungen koppelbar.
- Sowohl das Pumpmodul als auch die Infusionspumpe sind besonders einfach bedienbar und anwenderfreundlich.
- Das System ermöglicht gering wie auch hohe Förderraten jeweils bei genauer Dosierung.
- Das System ist absolut steril.

Der Antrieb für die Kolben-Zylinder-Einheit kann mit einer nicht dargestellten Drehüberwachung ausgerüstet sein. Hierdurch ist möglich, Störungen beim Zuführen des Fluids zu einem Patienten festzustellen, zum Beispiel einen Verschluss im Auslauf. Derartige Störungen können über Abweichungen des Antriebsverhaltens von üblichen Werten erkannt werden, zum Beispiel über ein Blockieren oder Verlangsamen des Motors. Mittels einer solchen Drehüberwachung kann auf eine Verwendung von ansonsten erforderlichen teuren Drucksensoren verzichtet werden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine perspektivische Darstellung einer Infusionspumpe nach der Erfindung mit eingesetztem Pumpmodul;
Fig. 2 eine perspektivische Darstellung einer erfindungsgemäßen Infusionspumpe mit eingesetztem Pumpmodul bei teilweise freigeschnittenem Pumpengehäuse;
Fig. 3 eine perspektivische Darstellung eines erfindungsgemäßen Pumpmoduls mit angeschlossenem Schlauch;
Fig. 4 eine perspektivische Darstellung einer Infusionspumpe sowie eines Pumpmoduls nach der Erfindung beim Einsetzen des Pumpmoduls in die Pumpe;
Fig. 5 eine detaillierte Ansicht eines Pumpmoduls mit integrierter Schiebeklemme;
Fig. 6 ein Pumpmodul mit daran angeschlossener Tropfkammer;
Fig. 7 ein Pumpmodul mit daran angeschlossenem Spike;
Fig. 8 ein Pumpmodul mit daran angeschlossenem Luer-Lock-Innenkegel;
Fig. 9 das Pumpmodul mit dem Spike aus Fig. 7 in die erfindungsgemäße Infusionspumpe eingesetzt;
Fig. 10 eine Ansicht eines Schnitts der Kolben-Zylinder-Einheit in axialer Richtung und
Fig. 11 eine Ansicht auf eine Ausführungsform des Pumpmoduls nach der Erfindung.

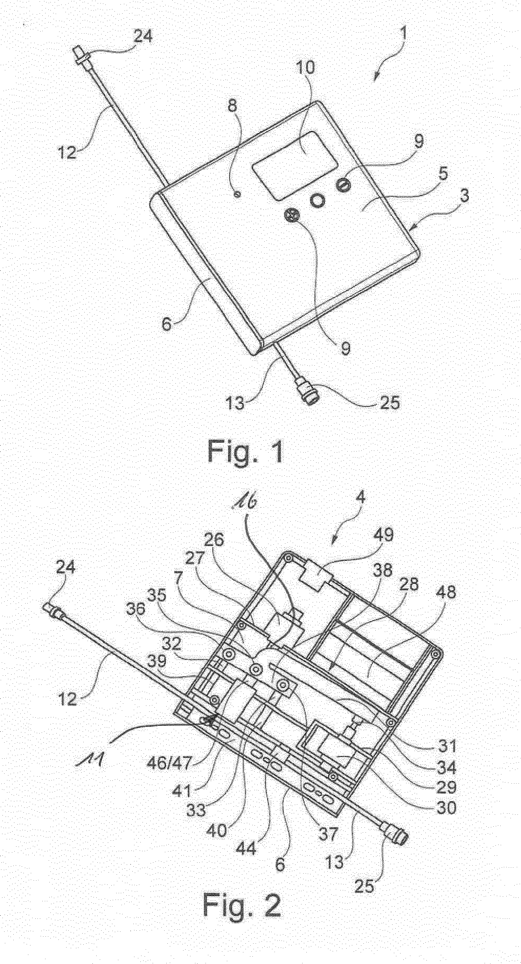

Figur 1 zeigt ein Ausführungsbeispiel einer Infusionspumpe 1 nach der Erfindung mit eingesetztem Pumpmodul 2. Die Pumpe 1 weist ein Gehäuse 3 auf, das im Wesentlichen bevorzugt aus einem Gehäuseunterteil 4 mit einem Gehäusedeckel 5 und einer Verschlussklappe 6 besteht. Das Gehäuseunterteil 4 und der Gehäusedeckel 5 sind miteinander verbindbar, zum Beispiel über in den Figuren nicht dargestellte Rastverbindung oder Verschraubungen, und bilden im zusammengefügten Zustand das Gehäuse 3 aus. Das Gehäuseunterteil 4 weist einen Gehäuseboden 7 auf. Im oder am Gehäusedeckel 5 sind im nachfolgenden beschriebene Einheiten/Elemente der Pumpe 1 angeordnet:
- Anzeigeelemente 8,
- Bedienungselemente 9 und ein
- Anzeigedisplay 10.

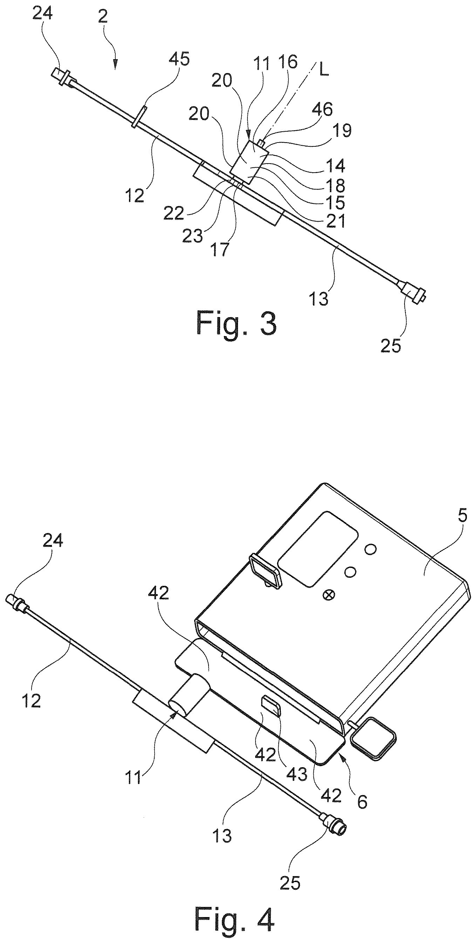

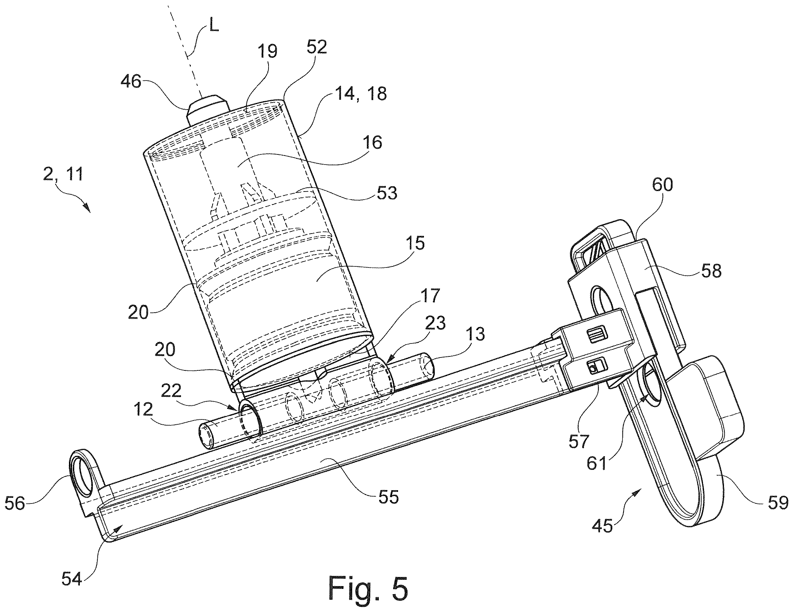

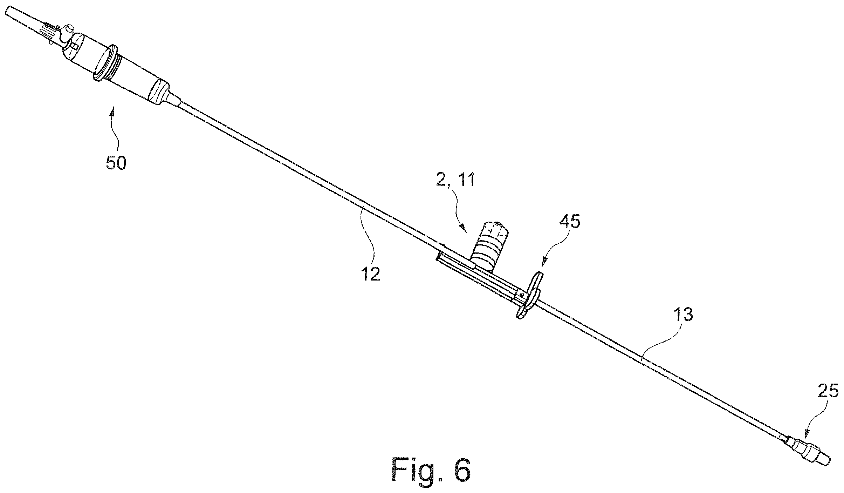

Das Pumpmodul 2 ist einzeln schematisch in Figur 3 gezeigt. Eine detailliertere Ausführungsform des Pumpmoduls 2 findet sich in Figur 5 wieder. Auch in den Figuren 6 bis 8 ist das Pumpmodul 2 dargestellt. Es weist eine Kolben-Zylinder-Einheit 11, einen ersten elastisch verformbaren Schlauchabschnitt 12 und einen zweiten elastisch verformbaren Schlauchabschnitt 13 auf. Das Pumpmodul 2 ist als Einmalartikel (Single Use Artikel, Wegwerfartikel) ausgebildet. Die Schlauchabschnitte 12, 13 können insbesondere als PVC-Schlauch ausgebildet sein.

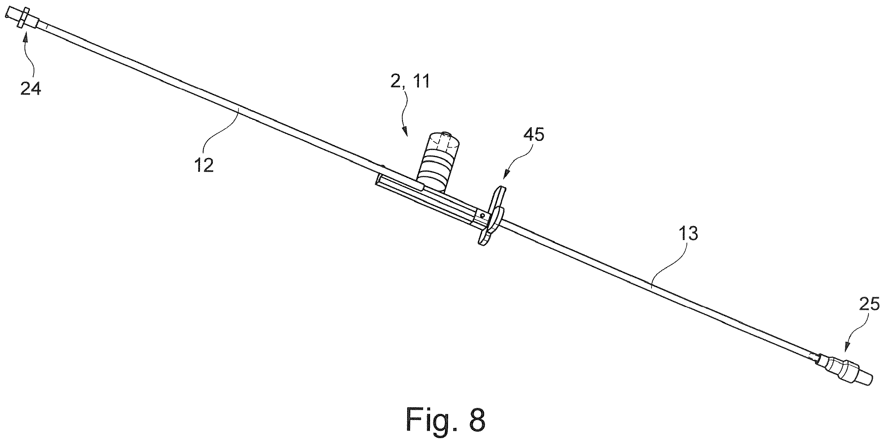

Die Kolben-Zylinder-Einheit 11 hat einen Zylinder 14, der insbesondere aus Kunststoff bestehen und/oder als Spritzgussteil hergestellt sein kann, und einen darin reziprok bewegbaren Kolben 15, der ebenfalls insbesondere aus Kunststoff bestehen und/oder als Spritzgussteil hergestellt sein kann. Der Kolben 15 ist auf/an einer Kolbenstange 16 angeordnet und zu dieser zumindest in axialer Richtung L lagefixiert. Der Zylinder 14 weist eine Bodenwand 17, eine Umfangswand 18 und eine Deckelwand 19 auf. Die Kolbenstange 16 ragt durch eine in der Deckelwand 19 ausgebildete Öffnung in axialer Richtung L aus dem Zylinder 14 heraus. Der Kolben 15 ist bevorzugt mittels beiderseits daran angeordneten Dichtungen 20 gegenüber der Umfangswand 18 abgedichtet, so dass die Kolben-Zylinder-Einheit 11 einen Fluidraum / Dosierraum 21 aufweist. In der Bodenwand 17 sind eine Einlaufbohrung 22 und eine Auslaufbohrung 23 ausgebildet. Der Fluidraum 21 ist über die Einlaufbohrung 22 strömungstechnisch mit dem ersten Schlauchabschnitt 12 und über die Auslaufbohrung 23 strömungstechnisch mit dem zweiten Schlauchabschnitt 13 verbunden. Die Schlauchabschnitte 12, 13 können insbesondere an den Zylinder 14 angeformt sein. Sie können insbesondere wie übliche Infusionsschläuche ausgebildet sein. Der erste Schlauchabschnitt 12 ist in der Ausführungsform aus Figur 3 an seinem von der Kolben-Zylinder-Einheit 11 abgewandten Ende mit einem Einlass-Luer-Lock-Anschluss 24 versehen, der vorliegend, wie auch in Fig. 8 als Luer-Lock-Innenkegel ausgebildet ist. Der zweite Schlauchabschnitt 13 ist in der Ausführungsform aus Figur 3 sowie in den Ausführungsformen in den Figuren 6 bis 9 an seinem von der Kolben-Zylinder-Einheit 11 abgewandten Ende mit einem Auslass-Luer-Lock-Anschluss 25 versehen, der vorliegend als Luer-Lock-Außenkegel ausgebildet ist. Damit ist das Pumpmodul 2 einfach an übliche Infusionseinheiten anzuschließen.

Die Figuren 2 und 5 zeigen, dass die Pumpe 1 einen (pumpeneigenen) Antrieb 26 für die Kolben-Zylinder-Einheit 11 aufweist, hier in Form eines Linearmotors 26. Der Motor 26 ist kongruent zur Längsachse L der Kolbenstange 16 (der Kolben-Zylinder-Einheit 11) angeordnet, so dass Kräfte zum reziproken Antreiben des Kolbens 15 zentral und ohne Verwindungen / Querkräfte übertragen werden können. Der Antrieb 26 ist über eine Montage in einer Zwischenwand 27 lagefixiert am Gehäuseunterteil 4 angeordnet. Die Kolbenstange 16 ist mittels eines daran angeordneten oder ausgebildeten Kuppelelements 46 und eines zum Eingriff damit ausgebildeten Gegenelements 47 mit dem Antrieb 26 verbindbar. Das Kuppelelement 47 dient demnach als Aufnahme für den Antrieb / Linearmotor 26.

Figur 2 zeigt des Weiteren, dass die Pumpe 1 eine (pumpeneigene) Steuereinheit 28 zumindest zum steuernden Zusammenquetschen der Schlauchabschnitte 12, 13 aufweist. Die Steuereinheit 28 ist wirktechnisch mit dem ersten Schlauchabschnitt 12 und dem zweiten Schlauchabschnitt 13 verbindbar und über eine weitere Zwischenwand 29 am Gehäuseunterteil 4 angeordnet. Sie hat eine mittels eines (angesteuerten/geregelten) Antriebs 30, hier eines Linearmotors 30, motorisch angetriebene Kipphebeleinheit mit einem Kipphebel 31 und zwei schwenkbar daran angeordneten Druckstempeln 32, 33 (Ventileinrichtungen). Der Kipphebel 31 ist über einen Mitnehmer 34 mit dem Linearmotor 30 verbunden und über ein Kipplager 35 um dessen in Figur 2 orthogonal zur Zeichnungsebene und in der Figur nicht gezeigte Kippachse schwenkbar gelagert. Die Druckstempel 32, 33 sind schwenkbar mittels Schwenklagern 36, 37 an einem Querarm 38 des Kipphebels 31 angeordnet und am Gehäuseunterteil 4 linearpositionierbar geführt. Sie sind mittels Druckfedern 39, 40 vorgespannt, derart, dass die Spannung der Druckfedern 39, 40 sie von den beiden Schlauchabschnitten 12, 13 fort drängt.

Figur 2 zeigt, dass die Druckstempel 32, 33 orthogonal zu den Schlauchabschnitten 12, 13 angeordnet sind. Sie sind des Weiteren in einer Richtung orthogonal zu den Schlauchabschnitten 12, 13 linearpositionierbar. Die Druckstempel 32, 33 sind derart ausgebildet und angeordnet und dazu vorgesehen, in einer ersten Kippstellung, die in Figur 2 gezeigt ist, den ersten Schlauchabschnitt 12 zusammen zu quetschen und den zweiten Schlauchabschnitt 13 freizugeben. In einer zweiten Kippstellung, die in keiner der Figuren gezeigt ist, ist der einlaufschlauchseitige Druckstempel 32 aus der in Figur 2 gezeigten Lage vom ersten Schlauchabschnitt 12 fort positioniert, also quasi in Richtung des Motors 26 verlagert, während der auslaufschlauchabschnittseitige Druckstempel 33 in Richtung des zweiten Schlauchabschnitts verlagert ist und diesen zusammenquetscht. In dieser Lage ist der erste Schlauchabschnitt 12 nicht mehr durch den Druckstempel 32 zusammengequetscht und sein Durchflussquerschnitt ist freigegeben. Der Linearmotor 30 ist in der in Figur 2 gezeigten Situation, in der der erste Schlauchabschnitt zusammengequetscht wird, ausgefahren. In der vorstehend beschriebenen und nicht dargestellten Funktionsstellung, in der der zweite Schlauchabschnitt 13 zusammengequetscht ist, ist er eingefahren. Durch reziprokes Ein-und Ausfahren des Motors 30 wird der Kipphebel 31 zu einer Kippbewegung um sein Lager 35 angeregt, durch die die beschriebene Verlagerung der Druckstempel 32, 33 bewirkt wird.

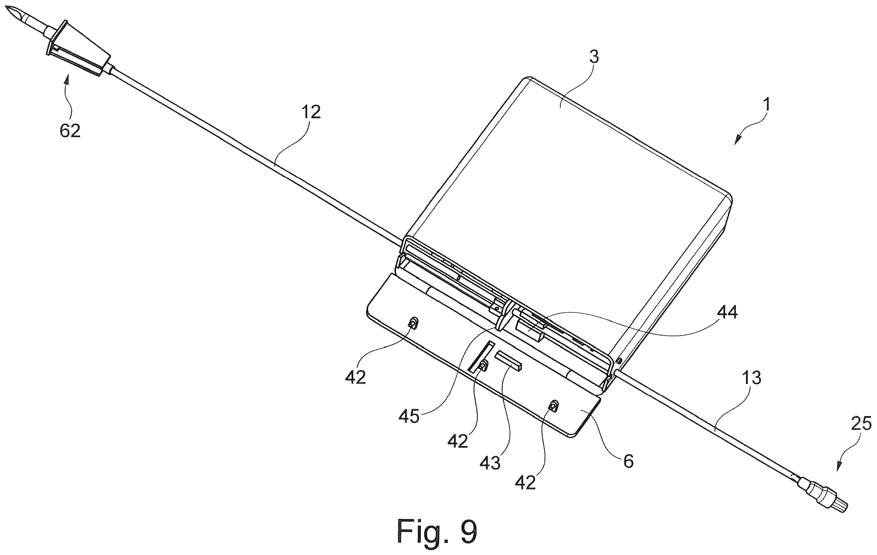

Aus den Figuren 1, 2, 4 und 9 ergibt sich, dass die Verschlussklappe 6 zwischen einer geöffneten Stellung (Figuren 4 und 9) und einer geschlossenen Stellung (Figuren 1 und 2) schwenkbar am Gehäuseunterteil 4 angeordnet ist. Sie ist mittels eines Verriegelungsmechanismus 41 am Gehäuse 3 verriegelbar und weist zu diesem Zweck mit dem Mechanismus 41 zusammenwirkende Riegelstifte 42 auf. Sie ist des Weiteren mit einem Positionierungszapfen 43 versehen, der mit dem zweiten Schlauchabschnitt 13 zusammenwirkt und sicherstellt, dass dieser bei geschlossener Verschlussklappe 6 bestimmungsgemäß an einem Luftsensor 44 platziert ist. Die Antriebe 26, 28 der Pumpe werden über eine Akku 48, der über einen USB-Anschluss 49 aufzuladen ist, energetisch versorgt.

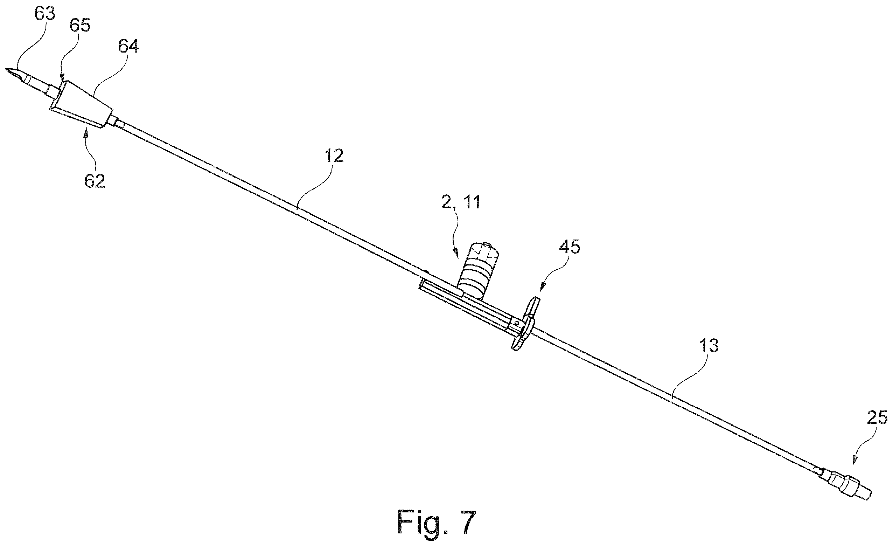

Zum Verwenden der Pumpe 1 öffnet ein Anwender zuerst die Verschlussklappe 6 der Pumpe 1, zum Beispiel indem er einen Spezialschlüssel seitlich in eine dafür vorgesehene Bohrung einführt und die Klappe 6 damit entriegelt. An dem Luer-Lock-Anschluss 24 des Pumpmoduls 2 aus den Figuren 1 bis 4 sowie 8 kann man beispielsweise ein Infusionsgerät anschließen. Weiterhin ist das Pumpenmodul 2 mit einer Tropfkammer 50 ausgestattet (Figur 6) oder mit einem Einstechdorn 63 ausgestattet (Figuren 7 und 9), die ebenfalls Bestandteile von als Einwegartikel ausgestalteten Infusionsleitungen sein können. Nachdem die Infusionsleitung einschließlich der beiden Schlauchabschnitte 12, 13 bis zum Auslass-Luer-Lock-Anschluss 25 / der Tropfkammer 50 / dem Einstechdorn 63 luftblasenfrei befüllt ist, wird der erste Schlauchabschnitt 12 (Figur 3) bzw. der zweite Schlauchabschnitt 13 (Figuren 5 bis 9) mittels einer Schiebeklemme 45 fluiddicht verschlossen. Danach schiebt der Anwender das Pumpmodul 2 in die dazu vorgesehene Aufnahme der Pumpe 1, bis das Kuppelelement 46 der Kolbenstange 16 mit dem Gegenelement 47 des Antriebs 26 hör-und/oder fühlbar einrastet und die Kolben-Zylinder-Einheit 11 mit dem Antrieb 26 verbunden ist. Nachfolgend wird die Verschlussklappe 6 verschlossen. Der im Gehäuseoberteil 5 befindliche Verschlussmechanismus 41 sorgt für eine sichere Arretierung der Klappe 6. Gleichzeitig wird der zweite Schlauchabschnitt 13 bestimmungsgemäß zum Luftsensor platziert, zum Beispiel an diesen gedrückt. Ein Erkennen und entsprechendes Kommunizieren von eventuell vorhandenen oder eingebrachten Luftblasen bei einer Infusion ist damit sichergestellt.

Jetzt gibt der Anwender die gewünschte Förderrate über die Anzeigeelemente 8 und die Bedienungselemente 9 ein. Deren Darstellung erfolgt über das Display 10, das nach einer Ausführungsform als Touch-Screen ausgebildet sein kann, so dass eine Eingabe auch über das Display 10 erfolgen kann (eine Bedienung über eine App im Handy oder Tablet ist außerdem möglich und im Rahmen der Erfindung). Unmittelbar nach Freigabe startet die Pumpe 1. Der Linearmotor 30 fährt aus der in Figur 2 gezeigten Stellung zurück und der Auslaufstempel 33 klemmt den zweiten Schlauchabschnitt 13 fluiddicht ab. Gleichzeitig öffnet der Einlaufstempel 32 den ersten Schlauchschnitt12. Danach fährt der Linearmotor 26 für den Kolben 15 aus der in Figur 2 gezeigten Stellung nach hinten und saugt Infusionslösung durch den ersten Schlauchabschnitt 12 in die Fluidkammer 21 der Kolben-Zylinder-Einheit 11 ein. Sobald die eingestellte Dosierung erreicht ist, fährt der Linearmotor 30 zurück nach vorn in die in Figur 2 gezeigte Stellung und der Einlaufstempel 32 verschließt den ersten Schlauchabschnitt 12 fluiddicht. Gleichzeitig öffnet der Auslaufstempel 33 den zweiten Schlauchabschnitt 13. Danach fährt der Linearmotor 26 bis zum Anschlag zurück nach vorn in die in Figur 2 gezeigte Stellung und drückt die in der Fluidkammer 21 befindliche Infusionsflüssigkeit in den zweiten Schlauchabschnitt13. Dieser Vorgang wird bis zum Erreichen der vorgegebenen Dosierung fortgeführt. Nach Beendigung der Infusion wird die Verschlussklappe 6 wieder mit dem Spezialschlüssel geöffnet. Außerdem kann mittels des Spezialschlüssels eine Entriegelung der Kupplung zwischen Antrieb 26 und Kolbenstange 16 erfolgen. Nun kann das Pumpmodul 2 aus dem Gehäuse 3 der Pumpe 1 entnommen werden.

Der detaillierte Aufbau des Pumpmoduls ist Figur 5 zu entnehmen.

Ein im Bereich der Deckelwand 19 (bzw. an dieser ausgeformter) befindlicher Ringwulst 52 stellt einen Axial-Anschlag für eine vorzugsweise integral mit der Kolbenstange 16 ausgestaltete Begrenzungsplatte 53 dar. So ist der maximale Hub der Kolben-Zylinder-Einheit 11 durch die Begrenzungsplatte 53 festgelegt und ein Herausfallen des Kolbens 15 aus dem Zylinder 14 formschlüssig vermieden. An der der Bodenwand 17 des Zylinders 14 zugewandten End-Seite der Kolbenstange 16 ist der Kolben 15 vorzugsweise als ein Leichtlaufkolben angeordnet/ausgebildet/fixiert. Der Kolben ist dabei weiter vorzugsweise als eine Kolbenhülse mit bestimmter Hülsenlänge oder als Doppelkolben mit zwei axialbeabstandeten Kolbenplatten (nicht weiter gezeigt) ausgebildet. Dieser weist an seinem distalen und an seinem proximalen Ende (oder distale und proximale Kolbenplatte jeweils) eine als bewegliche Dichtlippe ausgestaltete Dichtung 20 auf. Figur 5 gibt zu erkennen, dass jene Dichtungen 20 in einem bevorzugten Ausführungsbeispiel in Umfangsrichtung durchgehend als Ringdichtungen ausgestaltet sind, wodurch eine Unter- und Überdruckbewegung gewährleistet ist. Die Distanz entlang der Längsrichtung L zwischen der distalen und der proximalen Dichtung 20 des Kolbens 15 übersteigt in dem gezeigten Ausführungsbeispiel den Kolbenhub der Kolbenzylindereinheit 11. Somit ist garantiert, dass die Zylinderwandung des Fluidraums / Dosierraums 21 ausschließlich mit der einen, distalen Dichtung 20 in Kontakt ist, wodurch dessen Sterilität sichergestellt ist. Vorzugsweise beträgt der Überschuss zwischen der Distanz entlang der Längsrichtung L zwischen der distalen und der proximalen Dichtung 20 des Kolbens 15 und dem Kolbenhub mindestens 2 mm.

Das Pumpmodul 2 weist zudem eine Griffplatte 54 auf. Diese weist in ihrem Querschnittsprofil eine T-Form auf, wodurch sich eine Art Griffleiste oder Griffbereich 55 ergibt, mittels dem das Pumpmodul 2 leicht greifbar ist und in das Gehäuse 3 einsetzbar oder aus diesem herausnehmbar ist. Im Konkreten ist die Griffleiste 55 am distalen Ende der Kolben-Zylindereinheit 11 so angeordnet, dass sich die Griffleiste 55 quer zur Zylinder-Längsachse L, vorzugsweise senkrecht dazu erstreckt sowie weiter vorzugsweise parallel zu den Schlauchabschnitten 12, 13 verläuft. Die Griffleiste-/platte 54 ist an ihrem auf Seiten des ersten Schlauchabschnitts 12 zugewandten/befindlichen Ende mit einer Hülse oder Öse 56 versehen, die quer zur Griffleiste/-platte vorragt und deren Längsachse parallel zur Griffleiste/-platte verläuft. Die Hülse/Öse 56 ist vorzugsweise integral mit der Griffleiste/-platte 54 ausgestaltet und dient dazu, eine Infusionsleitung (z.B. den Schlauchabschnitt 12) zu fixieren, die durch sie hindurchgeführt wird. So definiert sie einen kreisrunden Hohlabschnitt, in dem die Infusionsleitung nach ihrem Einsetzen anliegt/eingeführt ist. An ihrem auf Seiten des zweiten Schlauchabschnitts 13 zugewandten/befindlichen Ende ist die Griffleiste/-platte 54 mit der Schiebklemme 45 mechanisch gekoppelt. Hierfür sind an der Griffleiste/- platte 54 Rastnocken oder Federzungen mit Rastvorsprüngen 57 angebracht/ausgebildet, wohingegen an der Schiebklemme 45 ein Einsteckschacht angeformt ist. Bei den Rastnocken 57 handelt es sich beispielsweise um quader- oder kugelförmige Vorstehelemente, die in entsprechende Gegenösen/Rücksprünge/Ausnehmungen im Einsteckschacht bzw. dessen Umfangswand der Schiebklemme 45 eingreifen, um diese positionsdefiniert mit der Griffleiste/-platte 54 zu koppeln. Bei jenem Verbinden handelt es sich demnach um einen Formschluss, der über ein Einschnappen der Gegenösen über die Rastnocken 57 realisiert ist.

Die Schiebklemme 45 weist einen starren Teil oder Rahmen 58 und einen beweglichen Teil oder Schieber 59 auf, der im starren Teil oder Rahmen 58 gelagert ist. Der Schieber 59 wie auch der Rahmen 58 sind jeweils mit einem Durchgangsloch/Öffnung ausgeformt, die in einer ersten Schiebeposition deckungsgleich positioniert sind und in einer zweiten Schiebeposition zueinander verschoben sind. Diese klemmen in der zweiten Schiebe-Position/Zustand, in dem die Pumpe 1 nicht fördert bzw. das Pumpmodul 2 nicht in das Gehäuse 3 eingesetzt ist, den ersten Schlauchabschnitt 13 derart ab, dass dessen Luftblasenfreiheit garantiert ist. In der ersten/weiteren Schiebeposition/Zustand wird der bewegliche Teil 59 relativ zu dem starren Teil 58 derart bewegt, dass die eine, erste Öffnung 60, die von dem starren Teil 58 ausgebildet ist, und die andere, zweite Öffnung 61, die von dem beweglichen Teil 59 der Schiebklemme 45 ausgebildet ist, bündig zueinander sind (sich überdecken) und so einen Fluidfluss durch den zweiten Schlauchabschnitt 13 freigeben, der durch die beiden Durchgangslöcher/Öffnungen hindurchgeführt ist.

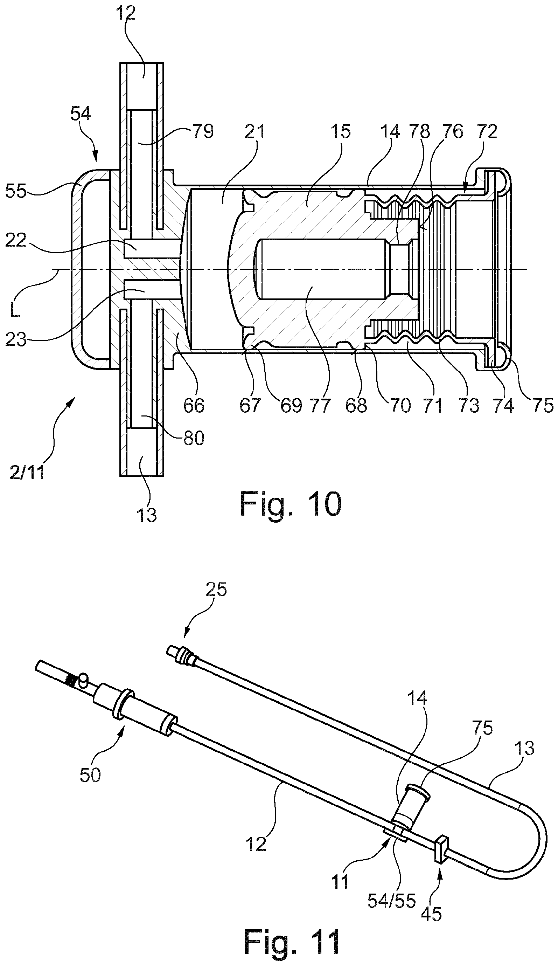

Der erfindungsgemäße Vorteil, dass die Infusionspumpe 1 für sämtliche Anwendungen einer Infusion, das heißt solche mit hohem Fördervolumen und solche mit hohen Genauigkeitsanforderungen, einsetzbar ist, wird weiter dadurch verstärkt, dass sämtliche Infusionssets an die Pumpe anschließbar sind. Beispielhaft hierfür offenbart Figur 6 die Kopplung des ersten Schlauchabschnitts 12 mit einer Tropfkammer 50. So ist es möglich, das Pumpmodul 2 mit einer Tropfkammer 50 zu koppeln und diese Einheit dann in das Gehäuse 3 einzusetzen. Die Tropfkammer 50, die im Regelfall Bestandteil jedes Infusionssystems ist, ist somit direkt stromabwärts zum Pumpmodul 2 anordenbar, wodurch die Kompaktheit des gesamten Infusionssystems wahlweise erhöht ist. Sie dient der regulierten Tropfenbildung der verabreichten Flüssigkeit sowie der Vermeidung einer Luftbläschenbildung. Figur 11 zeigt eine sehr ähnliche Ausführungsform, die sich dadurch unterscheidet, dass die Schiebeklemme 45 nicht direkt an der Kolben-Zylinder-Einheit 11 angeordnet, sondern separat ausgebildet ist.

In Figur 7 ist eine weitere Ausführungsform mit einem anderen Infusionsset dargestellt. So weist die Infusionsleitung hier stromabwärts zum Pumpmodul 2 einen Spike 62 auf. Dieser stellt das Verbindungsstück dar, das eine zuverlässige Verbindung zwischen einem externen Infusionsbehälter und den Infusionsleitungen herstellt. Der Spike 62 ist in einen Einstechdorn 63 und einen Haltesitz 64 einteilbar. Zwischen diesen ist ein Spikeanschlag 65 ausgebildet, der ein sicheres Sitzen des Spikes 62 ermöglicht.

In Figur 8 ist der erste Schlauchabschnitt 12, wie in Figur 3, mit dem Einlass-Luer-Lock-Anschluss / Luer-Lock-Innenkegel 24 versehen. Die Schiebeklemme 45 ist hierbei in einem solchen Zustand, dass sie ein Durchfließen des zweiten Schlauchabschnitts 13 zulässt. In diesem Zustand, in dem ein Durchfließen ermöglicht ist, ist das Pumpmodul 2 in das Gehäuse 3 eingesetzt, wie in Figur 2 und in Figur 9 dargestellt.

Figur 9 zeigt hierbei die Ausführungsform, in der der Spike 62 mit dem ersten Schlauchabschnitt 12 verbunden ist.

Figur 10 zeigt eine Ausführungsform der Kolben-Zylinder-Einheit 11 des Pumpmoduls 2 in einer entlang der axialen Richtung geschnittenen Ansicht. Sie umfasst einen Zylinder 14, der an seinem distalen Ende mit einem Zylinderkopf 66 versehen ist. In diesem sind ein Zulaufkanal 22 / eine Einlaufbohrung 22 und ein Ablaufkanal 23 / eine Auslaufbohrung 23 ausgebildet, die jeweils mit einem Anschlussstutzen 79, 80 zum Ankoppeln der Fluidzuleitung 12 bzw. der Fluidableitung 13 strömungstechnisch gekoppelt sind. An den Zylinderkopf 66 ist das Griffstück 54 mit dem Griffabschnitt 55 angeordnet, hier angeformt. Dieses dient der Handhabung der Kolben-Zylinder-Einheit 11 insbesondere bei einem Einführen in die Infusionspumpe 1.

Im Inneren des Zylinders 14 ist der Kolben 15 in axialer Richtung L translatorisch/hin-her bewegbar angeordnet. In der Wandung des Zylinders 14 sind eine umlaufende distale Ringnut 67 und eine umlaufende proximale Ringnut 68 ausgebildet. Diese dienen der Aufnahme einer distalen Kolbendichtung 69 bzw. einer proximalen Kolbendichtung 70. Die Kolbendichtungen 69, 70 sind hier als flexible Dichtlippen an den Kolben 15 angeformt, zum Beispiel mittels 2-K-Technik. Der Abstand zwischen den Ringnuten 67, 68 in Richtung der Längsachse L (axiale Richtung) gleicht dem Abstand zwischen den Kolbendichtungen 69, 70 in dieser Richtung, so dass die beiden Kolbendichtungen 69, 70 bei in einer Ruhe- oder Lagerstellung befindlichem Kolben 15 ohne große Vorspannung und Belastung durch die Wandung des Zylinders 14 in den entsprechenden Ringnuten 67, 68 aufgenommen und gelagert werden können. So ist gewährleistet, dass diese bei der Verwendung immer gut abdichten. Außerdem ist der Abstand zwischen den flexiblen Kolbendichtungen 69, 70 im vorliegenden Ausführungsbeispiel etwa 2 mm größer als der Hub der Kolbenpumpe, also als der Hub des Kolbens 15 im Zylinder 14. Dadurch ist sichergestellt, dass der mit der Infusionslösung in Kontakt gelangende Bereich immer absolut steril ist.

In der Kolben-Zylinder-Einheit 11 ist derart eine strömungstechnisch mit der Fluidzuleitung 12 und der Fluidableitung 13 verbundene Förderkammer 21 ausgebildet, die durch die Wandungen des Zylinders 14, den darin angeordneten Kolben und die den Kolben gegenüber dem Zylinder abdichtende distale Kolbendichtung 67 begrenzt ist. Ein auf der der Förderkammer 21 gegenüberliegenden Seite der Kolbendichtung 67 befindlicher Hubraum 71 ist mittels eines darin angeordneten elastischen Dichtelements 72 gegenüber der Umgebung steril abgedichtet. Das Dichtelement 72 ist einerseits am proximalen Ende des Kolbens und andererseits am proximalen Ende des Zylinders angeordnet und ragt nicht über das proximale Ende des Zylinders 14 hinaus. Es ist außerdem in Form einer topfförmigen Membran mit einem sich innerhalb des Zylinders 14 in Wesentlichen in axialer Richtung erstreckenden Faltenbalg 73 und einem proximalen ringförmigen Koppelabschnitt 74 ausgebildet. Der Faltenbalg 73 ist mit seiner dem Kolben 14 zugewandten distalen Seite dichtend stirnseitig des Kolbens 14 mit diesem verbunden, hier stoffschlüssig verbunden. Der ringförmigen Koppelabschnitt 74 ist mittels einer endseitigen Bördelung 75 bzw. eines Bördelrandes 75 mit dem Zylinder 14 fest und dicht verbunden. Anders als die in Figur 5 gezeigte Ausführungsform ist der Kolben 15 an seiner proximalen Stirnfläche 76 mit einer Kolbenstangenaufnahme 77 in Form eines zentralen Sacklochs 77 versehen, das in axialer Richtung L in die proximale Stirnfläche 76 des Kolbens 15 eingebracht ist und eine innenliegende Raststruktur 78 hier in Form eines Fixierrings 78 zur rastenden Aufnahme einer Kolbenstange 16 des Antriebsmechanismus aufweist.

Zur Verwendung führt ein Anwender die Kolben-Zylinder-Einheit 11 des blasenfrei mit Infusionslösung befüllten Pumpmoduls 2 in die dafür vorgesehene Vorrichtung der Infusionspumpe 1 ein. Dabei wird die Kolbenstange 16 in die Kolbenstangenaufnahme 77 des Kolbens 15 eingeführt und rastet in die Raststruktur 78 ein. Der Anwender stellt eine bestimmte Förderrate an der Infusionspumpe 1 ein und startet das System. Die Kolbenstange 16 bewegt nun den Kolben 15 beispielsweise mit einen Hub von 10 mm hin und her und fördert Infusionslösung in Richtung Patient. Der größere Abstand der Kolbendichtungen 69, 70, beispielsweise ein Abstand von 12 mm, gewährleistet, dass der zwischen diesen liegende Bereich immer steril bleibt. Durch das Dichtelement 72 wird außerdem der proximale Bereich des Zylinders 14 steril gehalten.

### Bezugszeichenliste

- 1: Infusionspumpe
- 2: Pumpmodul
- 3: Gehäuse
- 4: Gehäuseunterteil
- 5: Gehäusedeckel
- 6: Verschlussklappe
- 7: Gehäuseboden
- 8: Anzeigeelemente
- 9: Bedienungselemente
- 10: Anzeigedisplay
- 11: Kolben-Zylinder-Einheit
- 12: Fluidzuleitung, erster Schlauchabschnitt
- 13: Fluidableitung, zweiter Schlauchabschnitt
- 14: Zylinder
- 15: Kolben
- 16: Kolbenstange
- 17: Bodenwand
- 18: Umfangswand
- 19: Deckelwand
- 20: Dichtung
- 21: Fluidraum / Dosierraum / Förderkammer
- 22: Einlaufbohrung, Zulaufkanal
- 23: Auslaufbohrung, Ablaufkanal
- 24: Einlass-Luer-Lock-Anschluss, Luer-Lock-Innenkegel
- 25: Auslass-Luer-Lock-Anschluss, Luer-Lock-Außenkegel
- 26: Antrieb, Linearmotor
- 27: Zwischenwand
- 28: Steuereinheit
- 29: Zwischenwand
- 30: Antrieb, Linearmotor
- 31: Kipphebel
- 32: Druckstempel
- 33: Druckstempel
- 34: Mitnehmer
- 35: Kipplager
- 36: Schwenklager
- 37: Schwenklager
- 38: Querarm
- 39: Druckfeder
- 40: Druckfeder
- 41: Verschlussmechanismus
- 42: Riegelstift
- 43: Positionierungszapfen
- 44: Luftsensor
- 45: Schiebeklemme
- 46: Kuppelelement
- 47: Gegenelement
- 48: Akku
- 49: USB-Schnittstelle
- 50: Tropfkammer
- 51: Spike
- 52: Ringwulst
- 53: Begrenzungsplatte
- 54: Griffplatte, Griffstück
- 55: Griffbereich, Griffabschnitt
- 56: Hülse
- 57: Rastnocken
- 58: starrer Teil der Schiebeklemme
- 59: beweglicher Teil der Schiebeklemme
- 60: erste Öffnung der Schiebeklemme
- 61: zweite Öffnung der Schiebeklemme
- 62: Spike
- 63: Einstechdorn
- 64: Haltesitz
- 65: Spikeanschlag
- 66: Zylinderkopf
- 67: Ringnut
- 68: Ringnut
- 69: distale Kolbendichtung
- 70: proximale Kolbendichtung
- 71: Hubraum
- 72: Dichtelement
- 73: Faltenbalg
- 74: Koppelabschnitt
- 75: Bördelung, Bördelrand
- 76: Stirnfläche
- 77: Kolbenstangenaufnahme, Sackloch
- 78: Raststruktur, Fixierring
- 79: Anschlussstutzen
- 80: Anschlussstutzen
- L: Längsrichtung, Achsrichtung

## Patentansprüche

1. Pumpmodul (2) für eine Infusionspumpe (1), das eine zur wahlweisen/wechselbaren Kopplung mit der Infusionspumpe (1) ausgebildete, separate Kolben-Zylinder-Einheit (11) zur Förderung von Fluid von einer Fluidzuleitung (12) in eine Fluidableitung (13) zu einem Patienten aufweist, wobei die Kolben-Zylinder-Einheit (11) eine strömungstechnisch mit der Fluidzuleitung (12) und der Fluidableitung (13) verbundene/verbindbare Förderkammer (21) aufweist, die durch einen Zylinder (14), einen darin in axialer Richtung L translatorisch/hin-her bewegbar angeordneten Kolben (15) und eine den Kolben (15) gegenüber dem Zylinder (14) gleitend dichtende Kolbendichtung (20, 69, 70) begrenzt ist,
wobei
die Kolbendichtung (20, 69, 70) einen sich längs des Kolbens (15) erstreckenden und diesen umgebenden Hüllenabschnitt (73) aufweist, der relativ zum Kolben (15) beweglich ausgebildet ist und lose zwischen einem Umfangs-/Wandabschnitt des Zylinders (14) und dem Kolben (15) gehalten und an einem der Förderkammer (21) abgewandten Ende oder Endbereich des Zylinders (14) an diesem dichtend fixiert ist, derart, dass der von der Kolbendichtung (20, 69, 70) bei der Hin-her-Bewegung des Kolbens (15) überlaufene Umfangs-/ Wandabschnitt des Zylinders (14) gegenüber der Umgebung steril abgedichtet ist, **dadurch gekennzeichnet, dass** die Kolben-Zylinder-Einheit (11) eine proximale Kolbendichtung (70) und eine distale Kolbendichtung (69) aufweist, die beide zwischen dem Kolben (15) und dem Zylinder (14) dichten und die Sterilität der Förderkammer (21) gewährleisten und dass der Zylinder (14) auf seiner dem Kolben (15) zugewandten Innenfläche mit einer umlaufenden Ringnut (67, 68) versehen ist, zur Aufnahme der Kolbendichtung (20, 69, 70) in einer Lagerstellung/Ruhestellung darin, insbesondere mit einer proximalen umlaufenden Ringnut (68) und einer distalen umlaufenden Ringnut (67) versehen ist, deren Abstand in Längsrichtung L voneinander gleich dem Abstand der proximalen Kolbendichtung (70) und der distalen Kolbendichtung (69) in Längsrichtung L voneinander ist.

2. Pumpmodul (2) nach Anspruch 1, **dadurch gekennzeichnet, dass** der auf der der Förderkammer (21) gegenüberliegenden Seite der Kolbendichtung (20, 69) befindliche Hubraum (71) mittels eines darin angeordneten, den Hüllenabschnitt bildenden, elastischen Dichtelements (72), insbesondere einer Membran (72), gegenüber der Umgebung steril abgedichtet ist, das einerseits am proximalen Ende des Kolbens (15), insbesondere der Kolbendichtung (20, 69), und andererseits am proximalen Ende des Zylinders (14) angeordnet ist, wobei das Dichtelement (72) vorzugsweise nicht über das proximale Ende des Zylinders (14) ragt.

3. Pumpmodul (2) nach Anspruch 1 oder 2, **dadurch gekennzeichnet, dass** das Dichtelement (72) einen als Faltenbalg (73) ausgebildeten und sich insbesondere in axialer Richtung erstreckenden Axialabschnitt aufweist, der insbesondere innerhalb des Zylinders (14) angeordnet ist.

4. Pumpmodul (2) nach einem der vorstehenden Ansprüche, **dadurch gekennzeichnet, dass** das Dichtelement (72) an seiner von Kolben (15) abgewandten Seite einen insbesondere ringförmigen Koppelabschnitt (74) zum dichtenden Anordnen an / Verbinden mit dem Zylinder (14) aufweist, der insbesondere mittels einer Bördelung (74) des Zylinders (14) mit diesem dicht verbunden ist, und/oder dass das Dichtelement (72) mit seiner dem Kolben (15) zugewandten Seite dichtend stirnseitig des Kolbens (15) mit diesem verbunden ist, beispielsweise stoffschlüssig verbunden ist, insbesondere einstückig mit dem Kolben (15) ausgebildet ist, wobei der Koppelabschnitt (74) insbesondere in Form einer Dichtplatte (74) ausgebildet ist.

5. Pumpmodul (2) nach einem der vorstehenden Ansprüche, **dadurch gekennzeichnet, dass** der Kolben (15) eine Koppelstruktur (77) zum lösbaren Kuppeln mit einem entsprechenden Kuppelelement (16) des Antriebsmechanismus (26) der Infusionspumpe (1) aufweist.

6. Pumpmodul (2) nach Anspruch 5, **dadurch gekennzeichnet, dass** die Koppelstruktur (77) radial innerhalb des Dichtelements (72), insbesondere radial innerhalb des als Faltenbalg (73) ausgebildeten Wandabschnitts des Dichtelements (72), ausgebildet ist.

7. Pumpmodul (2) nach Anspruch 5 oder 6, **dadurch gekennzeichnet, dass** die Koppelstruktur (77) ein Sackloch (77) aufweist, das in axialer Richtung L in die proximale Stirnfläche (76) des Kolbens (15) eingebracht ist und insbesondere eine innenliegende Raststruktur (78) zur rastenden Aufnahme einer Kolbenstange (16) des Antriebsmechanismus (26) aufweist.

8. Pumpmodul (2) nach Anspruch 1, **dadurch gekennzeichnet, dass** die proximale Kolbendichtung (70) und die distale Kolbendichtung (69) zueinander planparallel angeordnet sind und/oder der Abstand in Richtung der Längsachse L zwischen der proximalen Kolbendichtung (70) und der distalen Kolbendichtung (69) größer ist als der Förderhub der Kolben-Zylinder-Einheit (11) / des Kolbens (15) im Zylinder (14).

9. Pumpmodul (2) nach Anspruch 1 oder 8, **dadurch gekennzeichnet, dass** die proximale Kolbendichtung (70) und/oder die distale Kolbendichtung (69) einstückig mit dem Kolben (15) ausgebildet ist bzw. sind, insbesondere mittels 2-K-Technologie angeformt ist bzw. sind.

10. Pumpmodul (2) nach einem der vorstehenden Ansprüche, **dadurch gekennzeichnet, dass** der Zylinder (14) an seinem distalen Ende einen Zylinderkopf (66) aufweist, in dem zumindest ein Fluiddurchlass (22, 23) ausgebildet ist, um die Förderkammer (21) strömungstechnisch mit der Fluidzuleitung (12) und/oder mit der Fluidableitung (13) zu verbinden.

11. Pumpmodul (2) nach einem der vorstehenden Ansprüche, **dadurch gekennzeichnet, dass** distal an dem Zylinder (14), insbesondere an dessen Zylinderkopf (66), ein Griffstück (54, 55) angeordnet ist, zur Handhabung des Pumpmoduls (2) beim Koppeln und Entkoppeln mit der Infusionspumpe (1).

12. Infusionspumpe (1) zur Förderung von Fluid von einer Fluidquelle zu einem Patienten, umfassend ein Pumpmodul (2) nach einem der vorstehenden Ansprüche.

## Claims

1. Pump module (2) for an infusion pump (1), which has a separate piston-cylinder unit (11) adapted for selective/changeable coupling with the infusion pump (1) for delivering fluid from a fluid feed line (12) into a fluid drain line (13) to a patient, wherein the piston-cylinder unit (11) comprises a delivery chamber (21) fluidically connected/connectable to the fluid feed line (12) and the fluid drain line (13) and which is delimited by a cylinder (14), a piston (15) arranged therein so as to be movable translationally/forwards and backwards in the axial direction L, and by a piston seal (20, 69, 70) which slidably seals the piston (15) with respect to the cylinder (14),
wherein
the piston seal (20, 69, 70) comprises a sleeve portion (73) extending along and surrounding the piston (15), the sleeve portion (73) being formed to be movable relative to the piston (15) and being loosely held between a circumferential/wall portion of the cylinder (14) and the piston (15) and being sealingly fixed to an end or end region of the cylinder (14) facing away from the delivery chamber (21), such that
the peripheral circumferential/wall portion of the cylinder (14) passed by the piston seal (20, 69, 70) during the forwards/backwards movement of the piston (15) is sealed in a sterile manner with respect to the environment,
**characterized in that** the piston-cylinder unit (11) comprises a proximal piston seal (70) and a distal piston seal (69) which both seal between the piston (15) and the cylinder (14) and ensure the sterility of the delivery chamber (21), and that the cylinder (14) is provided on its inner surface facing the piston (15) with a circumferential annular groove (67, 68) for receiving the piston seal (20, 69, 70) in a storing position/rest position therein, in particular with a proximal circumferential annular groove (68) and a distal circumferential annular groove (67), the distance of which in the longitudinal direction L from each other is equal to the distance of the proximal piston seal (70) and the distal piston seal (69) from each other in the longitudinal direction L.

2. Pump module (2) according to claim 1, **characterized in that** the piston displacement (71) located on the side of the piston seal (20, 69) opposite the delivery chamber (21) is sealed in a sterile manner by means of an elastic sealing element (72), in particular a membrane (72), which is arranged therein and forms the sleeve portion, which is arranged on the one hand at the proximal end of the piston (15), in particular the piston seal (20, 69), and on the other hand at the proximal end of the cylinder (14), wherein the sealing element (72) preferably does not project beyond the proximal end of the cylinder (14).

3. Pump module (2) according to claim 1 or 2, **characterized in that** the sealing element (72) has an axial section which is designed as bellows (73) and which extends in particular in the axial direction and is arranged in particular inside the cylinder (14).

4. Pump module (2) according to one of the preceding claims, **characterized in that** the sealing element (72) has, on its side facing away from the piston (15), an in particular annular coupling portion (74) for sealing arrangement on/connection to the cylinder (14), said coupling portion (74) being sealingly connected to the cylinder (14) in particular by means of a border (74) of the cylinder (14), and/or **in that** the sealing element (72), with its side facing the piston (15), is sealingly connected to the front side of the piston (15), for example is connected in a material-locking manner, in particular in one piece with the piston (15), wherein the coupling portion (74) is in particular formed as a sealing plate (74).

5. Pump module (2) according to one of the preceding claims, **characterized in that** the piston (15) comprises a coupling structure (77) for releasable coupling with a corresponding coupling element (16) of the drive mechanism (26) of the infusion pump (1).

6. Pump module (2) according to claim 5, **characterized in that** the coupling structure (77) is formed radially inside the sealing element (72), in particular radially inside the wall portion of the sealing element (72) formed as bellows (73).

7. Pump module (2) according to claim 5 or 6, **characterized in that** the coupling structure (77) has a blind hole (77) which is introduced in the axial direction L into the proximal front surface (76) of the piston (15) and in particular has an internal latching structure (78) for latching reception of a piston rod (16) of the drive mechanism (26).

8. Pump module (2) according to claim 1, **characterized in that** the proximal piston seal (70) and the distal piston seal (69) are arranged plane-parallel to each other and/or the distance in the direction of the longitudinal axis L between the proximal piston seal (70) and the distal piston seal (69) is greater than the delivery stroke of the piston-cylinder unit (11)/the piston (15) in the cylinder (14).

9. Pump module (2) according to claim 1 or 8, **characterized in that** the proximal piston seal (70) and/or the distal piston seal (69) is or are formed in one piece with the piston (15), in particular by means of 2-K technology.

10. Pump module (2) according to one of the preceding claims, **characterized in that** the cylinder (14) has at its distal end a cylinder head (66) in which at least one fluid passage (22, 23) is formed in order to fluidically connect the delivery chamber (21) to the fluid feed line (12) and/or to the fluid drain line (13).

11. Pump module (2) according to one of the preceding claims, **characterized in that** a handle piece (54, 55) is arranged distally on the cylinder (14), in particular on its cylinder head (66), for handling the pump module (2) during coupling and uncoupling with the infusion pump (1).

12. An infusion pump (1) for delivering fluid from a fluid source to a patient comprising a pump module (2) according to one of the preceding claims.

## Revendications

1. Module de pompe (2) pour une pompe à perfusion (1), qui présente une unité piston-cylindre (11) séparée conçue pour être accouplée de manière sélective/interchangeable à la pompe à perfusion (1) pour le transport de fluide d'une conduite d'alimentation de fluide (12) dans une conduite de décharge de fluide (13) vers un patient, dans lequel l'unité piston-cylindre (11) présente une chambre de distribution (21) qui est reliée/peut être reliée fluidiquement à la conduite d'alimentation de fluide (12) et à la conduite de décharge de fluide (13), laquelle chambre est délimitée par un cylindre (14), par un piston (15) disposé dans celui-ci de manière à pouvoir être animé d'un mouvement de translation/de va-et-vient dans la direction axiale L et par un joint de piston (20, 69, 70) fermant de manière coulissante le piston (15) par rapport au cylindre (14),
dans lequel le joint de piston (20, 69, 70) comprend une section de manchon (73) s'étendant le long du piston (15) et entourant celui-ci, qui est conçue pour être mobile par rapport au piston (15) et est maintenue de manière lâche entre une section périphérique/de paroi du cylindre (14) et le piston (15) et est fixée à une extrémité opposée à la chambre de distribution (21) ou une zone d'extrémité du cylindre (14) de manière étanche à celui-ci, de telle sorte que la section périphérique/de paroi du cylindre (14) dépassée par le joint de piston (20, 69, 70) lors du mouvement de va-et-vient du piston (15) est étanchéifiée de façon stérile par rapport à l'environnement, **caractérisé en ce que** l'unité piston-cylindre (11) présente un joint de piston proximal (70) et un joint de piston distal (69) qui assurent conjointement l'étanchéité entre le piston (15) et le cylindre (14) et garantissent la stérilité de la chambre de distribution (21) et **en ce que** le cylindre (14) est muni sur sa surface intérieure dirigée vers le piston (15) d'une rainure annulaire périphérique (67, 68), pour la réception du joint de piston (20, 69, 70) dans une position de stockage/repos, en particulier d'une rainure annulaire périphérique proximale (68) et d'une rainure annulaire périphérique distale (67) dont la distance entre elles dans la direction longitudinale L est égale à la distance entre le joint de piston proximal (70) et le joint de piston distal (69) dans la direction longitudinale L.

2. Module de pompe (2) selon la revendication 1, **caractérisé en ce que** la zone de course (71) se trouvant sur la face opposée à la chambre de distribution (21) du joint de piston (20, 69) est étanchéifiée de façon stérile par rapport à l'environnement au moyen d'un élément d'étanchéité (72), en particulier d'une membrane (72), élastique, formant la section de manchon, disposé à l'intérieur de la zone de course, lequel élément d'étanchéité est agencé d'une part à l'extrémité proximale du piston (15), en particulier du joint de piston (20, 69), et d'autre part à l'extrémité proximale du cylindre (14), dans lequel l'élément d'étanchéité (72) ne dépasse pas de préférence de l'extrémité proximale du cylindre (14).

3. Module de pompe (2) selon la revendication 1 ou 2, **caractérisé en ce que** l'élément d'étanchéité (72) présente une section axiale conçue sous forme de soufflet (73) et s'étendant particulièrement dans la direction axiale, laquelle est agencée en particulier à l'intérieur du cylindre (14).

4. Module de pompe (2) selon l'une quelconque des revendications précédentes, **caractérisé en ce que** l'élément d'étanchéité (72) présente sur sa face opposée au piston (15) une section d'accouplement (74) en particulier annulaire à des fins d'agencement sur / de liaison étanche avec le cylindre (14), qui est reliée de manière étanche à celui-ci en particulier au moyen d'un bord rabattu (74) du cylindre (14), et/ou **en ce que** l'élément d'étanchéité (72) est relié de manière étanche à celui-ci par sa face dirigée vers le piston (15) sur la face frontale du piston (15), est reliée par exemple par complémentarité de matière, est conçue en particulier d'un seul tenant avec le piston (15), dans lequel la section d'accouplement (74) est conçue en particulier sous forme d'une plaque étanche (74).

5. Module de pompe (2) selon l'une quelconque des revendications précédentes, **caractérisé en ce que** le piston (15) présente une structure d'accouplement (77) à des fins d'accouplement amovible avec un élément d'accouplement (16) correspondant du mécanisme d'entraînement (26) de la pompe à perfusion (1).

6. Module de pompe (2) selon la revendication 5, **caractérisé en ce que** la structure d'accouplement (77) est conçue radialement à l'intérieur de l'élément d'étanchéité (72), en particulier radialement à l'intérieur de la section de paroi conçue sous forme de soufflet (73) de l'élément d'étanchéité (72).

7. Module de pompe (2) selon les revendications 5 ou 6, **caractérisé en ce que** la structure d'accouplement (77) présente un trou borgne (77) qui est monté dans la direction axiale L dans la surface frontale proximale (76) du piston (15) et présente en particulier une structure d'encliquetage intérieure (78) à des fins de logement par encliquetage d'une tige de piston (16) du mécanisme d'entraînement (26).

8. Module de pompe (2) selon la revendication 1, **caractérisé en ce que** le joint de piston proximal (70) et le joint de piston distal (69) sont agencés dans des plans mutuellement parallèles et/ou la distance en direction de l'axe longitudinal L entre le joint de piston proximal (70) et le joint de piston distal (69) est supérieure à la course d'avance de l'unité piston-cylindre (11) / du piston (15) dans le cylindre (14).

9. Module de pompe (2) selon la revendication 1 ou 8, **caractérisé en ce que** le joint de piston proximal (70) et/ou le joint de piston distal (69) est et/ou sont conçu(s) d'une seul tenant avec le piston (15), en particulier est et/ou sont formé(s) au moyen de la technologie 2K.

10. Module de pompe (2) selon l'une quelconque des revendications précédentes, **caractérisé en ce que** le cylindre (14) présente à son extrémité distale une tête de cylindre (66) dans laquelle est conçu au moins un passage de fluide (22, 23) pour relier fluidiquement la chambre de distribution (21) à la conduite d'alimentation de fluide (12) et/ou à la conduite de décharge de fluide (13).

11. Module de pompe (2) selon l'une quelconque des revendications précédentes, **caractérisé en ce qu'**une poignée (54, 55) est agencée distalement au niveau du cylindre (14), en particulier au niveau de sa tête de cylindre (66), à des fins de manipulation du module de pompe (2) lors de l'accouplement et du désaccouplement de la pompe à perfusion (1).

12. Pompe à perfusion (1) pour le transport de fluide d'une source de fluide vers un patient, comprenant un module de pompe (2) selon l'une quelconque des revendications précédentes.
